# EUROPEAN PATENT SPECIFICATION

(11) **EP 3 201 229 B1**
(45) Date of publication and mention of the grant of the patent: **09.09.2020**
(21) Application number: 15770951.0
(22) Date of filing: 29.09.2015
(51) Int. Cl.: C07K 16/22, A61K 39/00

(54) **A METHOD OF TREATING JOINT DISEASE**
VERFAHREN ZUR BEHANDLUNG VON ENTZÜNDLICHEN GELENKERKRANKUNGEN
PROCÉDÉ DE TRAITEMENT D'UNE MALADIE INFLAMMATOIRE DES ARTICULATIONS

(30) Priority: 30.09.2014 EP 14187166
(43) Date of publication of application: 09.08.2017
(73) Proprietor: VIB VZW, 9052 Gent (BE); Universiteit Gent, 9000 Gent (BE)
(72) Inventor: ELEWAUT, Dirk, B-9070 Heusden (BE); LAMBRECHT, Stijn, B-9860 Landskouter (BE)
(86) International application number: PCT/EP2015/072473
(87) International publication number: WO 2016/050796

(56) References cited:
- WO-A1-2014/100689
- JOHNEN HEIKO ET AL: "The in vivo biology of the TGF-b superfamily cytokine MIC-1 and its role in mouse models of rheumatoid arthritis", IMFLAMMATION RESEARCH, BIRKHAEUSER VERLAG, BASEL, CH, vol. 54, no. Suppl.2, 1 August 2005 (2005-08-01), page S122, XP009182901, ISSN: 1023-3830
- S. LAMBRECHT ET AL: "Reduced levels of the TGFb family member GDF15 in spondyloarthritis versus other rheumatic diseases", ANNALS OF THE RHEUMATIC DISEASES, vol. 70, no. Suppl 2, 22 February 2011 (2011-02-22), pages A88-A88, XP055173041, ISSN: 0003-4967, DOI: 10.1136/ard.2010.149021.15
- LAMBRECHT ET AL.: "GDF15, a distinct TGF-beta family member, is differentially regulated in spondyloarthritides compared to other rheumatic diseases", ANNALS OF TEH RHEUMATIC DISEASES, vol. 71, no. Suppl 1, 2012, pages A92-A93, XP002751865,
- BREIT SAMUEL N ET AL: "The TGF-[beta] superfamily cytokine, MIC-1/GDF15: a pleotrophic cytokine with roles in inflammation, cancer and metabolism", GROWTH FACTORS, HARWOOD ACADEMIC PUBLISHERS GMBH, XX, vol. 29, no. 5, 1 October 2011 (2011-10-01), pages 187-195, XP008152690, ISSN: 0897-7194
- Perez De La Lastra ET AL: "Epitope mapping of 10 monoclonal antibodies against the pig analogue of human membrane cofactor protein (MCP)", Immunology, vol. 96, no. 4, 1 April 1999 (1999-04-01), pages 663-670, XP55572134, GB ISSN: 0019-2805, DOI: 10.1046/j.1365-2567.1999.00732.x

## Description

### FIELD OF THE INVENTION

The invention relates to GDF15 antibodies for use in the treatment of rheumatoid arthritis, spondyloarthritis, psoriatic arthritis and osteoarthritis involving the inhibition or inactivation of growth differentiation factor 15 (GDF15) in an individual.

### BACKGROUND TO THE INVENTION

Growth Differentiation Factor (GDF15) is a member of the TGFβ-superfamily. GDF15, or Macrophage Inhibitory Cytokine (MIC-1), was originally identified as a factor secreted by activated macrophages [1]. Sequence analysis has shown that GDF15 is a more distant member of the TGFβ-family [2]: GDF15 shares it homology based on the typical TGFβ cysteine domains, but shares even less than 30% sequence homology to other family members such as TGFβ1, GDF5 and BMPs. The protein is expressed in a variety of tissues. High levels of GDF15 are detected in the placenta and to a lower extent in kidney, pancreas and prostate tissue [3]. GDF15 expression is usually low in resting tissues but may be increased following an adaptive stress response to diverse cellular stress signals, such as hypoxia and anoxia, inflammation, short-wavelength light exposure and tissue injuries (reviewed in [4]). In cardiovascular research, GDF15 emerged as an independent predictor of mortality [5, 6]. In this perspective, elevated GDF15 levels are considered as an endogenous protective mechanism, trying to limit cardiovascular damage [7]. Recently, it was demonstrated that GDF15 is involved in CCR-2 mediated chemotaxis [8]. Furthermore, WO2005/099746 describes that increased GDF15 expression in cancer patients is associated with cachexia and that by inhibiting that expression, it would be possible to reverse or reduce the severity of weight loss.

Today, surprisingly little is known about GDF15 serum levels in patients with rheumatic diseases. GDF15 was previously shown to be elevated in serum of RA-patients compared to healthy controls [9]. Also, Lambrecht et al (2011) Ann. Rheum. Dis. 70 (suppl. 2): A88, Abstract no. 205 and Lambrecht et al (2012) Ann. Rheum. Dis. 71 (suppl. 1): A93, abstract No. 18 disclose that patients suffering from spondyloarthritis had GDF15 serum levels near normal whereas patients with inflammatory rheumatic disease had elevated levels. However, the correlation between GDF15 concentrations and disease severity observed by Brown et al. was based on a very specific population: GDF15 levels were compared between very severe RA patients (patients that classified to undergo hematopoietic stem cell transplantation, having severe refractory disease) and an outpatient RA-group [9]. Breitt et al., 2011[25] and Johnen et al (2005) Inflamm. Res. 54 (suppl. 2): S122, abstract No. 5025, suggest a protective anti-inflammatory effect of GDF15 in rheumatoid arthritis. It is generally known that growth factors of the TGFβ superfamily are involved in the regulation of immune processes and therefore potentially implicated in the pathogenesis of several rheumatic diseases. Furthermore, it was previously shown that GDF15 induces endochondral ossification [3] and thus might contribute to arthritis associated bone formation. In addition, GDF15 also influences osteoclast metabolism. However, both osteoclast induction [21] and inhibition [22] by GDF15 were reported.

Currently, it is not clear which are the initial triggers of elevated GDF15 levels in SpA and RA patients. This might be associated with earlier described altered macrophage activation patterns in RA compared to SpA patients [23]. Hence GDF15, also known as Macrophage Inhibitory Cytokine (MIC-1), was previously associated with macrophage activation [24]. However, the true biological role of this protein in macrophage biology is not known. Recently, GDF15 has been shown to sensitize CCR2 mediated chemotactic responses, comprising an upregulation of CCR2 as well as its ligand MCP1 [8].

Clinically, joint inflammation is associated with joint stiffness, pain, weakness, and sometimes joint fatigue. Uniformly, the joint is tender and swollen, and often erythematous. Diagnosis of the inflammatory nature of the joint disease is frequently based upon this typical clinical presentation as well as upon radiographic examination and aspiration and examination of synovial joint fluid. Examination of joint fluid of an inflamed joint generally reveals elevation of various markers of inflammation, such as, leukocytes (including neutrophils), antibodies, cytokines, cell adhesion molecules, and complement activation products. Radiographic examination of affected joints generally reveals soft tissue swelling and/or erosive changes.

Rheumatoid arthritis (RA) is an autoimmune disease which causes chronic inflammation of the joints, the tissue around the joints, as well as other organs in the body. Spondyloarthropathy (SpA) is the name given to a group of chronic or long lasting diseases also called Spondyloarthritis or Spondylitis. Osteoarthritis (OA) (also known as degenerative arthritis, hypertrophic arthritis, or age-related arthritis) is a complex degenerative disease of the synovial joints. OA is characterized by loss and erosion of articular cartilage, subchondral sclerosis, and bone overgrowth (osteophytes). Inflammatory joint diseases are all characterized by inflammatory joint defects that can lead to severe, chronic pain and discomfort.

Treatment of inflammatory joint diseases has become much more efficient in the past decade by the introduction of "biologicals", e.g. anti-TNFa treatment. However, an important part of the patients (30-40%) eligible for biologics treatment, does not respond well to anti-TNF or becomes refractive within a short time frame. Moreover, treatment with anti-TNF (or other biologicals) has not shown to be highly efficient for preventing new bone formation, a typical feature of SpA with important clinical consequences. Finally, immunosuppression is an important side-effect of anti-TNF treatment. In conclusion, there is a clear unmet clinical need in this area for new treatment strategies of inflammatory joint disease that do not act directly on the immune-system, in contrast to all current biologics available.

In the present invention, evidence was obtained for a therapeutic role of GDF15 in joint inflammation and/or joint remodeling. Lack of expression of GDF15 shows a clear reduction in the severity of arthritis in experimental animal models and opens up a new therapeutic perspective which has been demonstrated for the first time in the present invention.

### BRIEF DESCRIPTION OF THE FIGURES

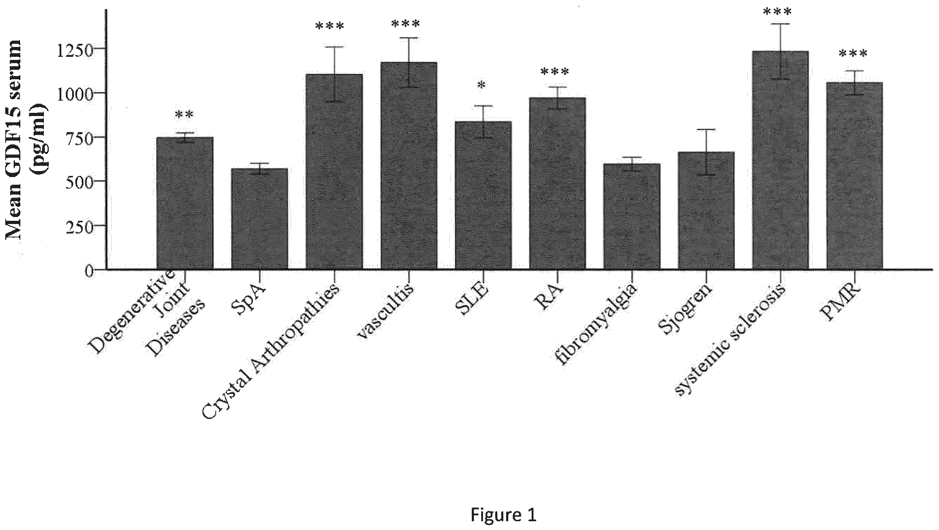

**Figure 1****:** Serum levels of GDF15 in several patient cohorts. GDF15 serum levels are significantly elevated in several inflammatory rheumatic diseases compared to SpA patients (* p<0,05; ** p<0,01; *** p<0,001). p-Values are the result of a Bonferroni's multiple comparison test on the log-transformed concentrations.

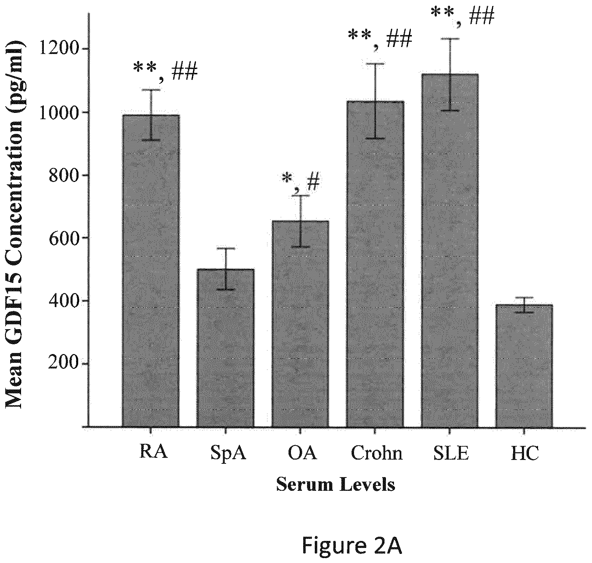

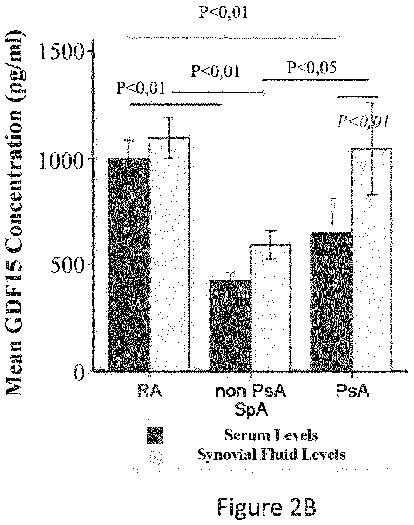

**Figure 2****: A:** Serum levels of GDF15 in several patient cohorts. GDF15 serum levels are significantly elevated in RA and SLE patients compared to SpA patients (* p<0,05, **, p<0,01) and to healthy controls (# p<0,05, ##, p<0,01). P-values are the result of an Bonferroni's multiple comparison test on the log-transformed concentrations. **B:** Serum levels (dark grey) and synovial fluid levels (light gray) of RA and non-psoriatic arthritis (non-PsA) SpA and psoriatic arthritis (PsA) patients. Synovial fluid levels are significantly higher compared to serum levels in PsA patients only (paired samples t-test on log transformed serum and synovial fluid concentrations of the same patient). Synovial fluid levels are, comparable to serum levels, significantly higher in RA patients compared non-PsA SpA patients (independent student's T-test on the log-transformed concentrations). P-values in italic are the result of a paired samples test, all other p-values are derived from an independent samples test. Bars represent the mean serum level ± SEM.

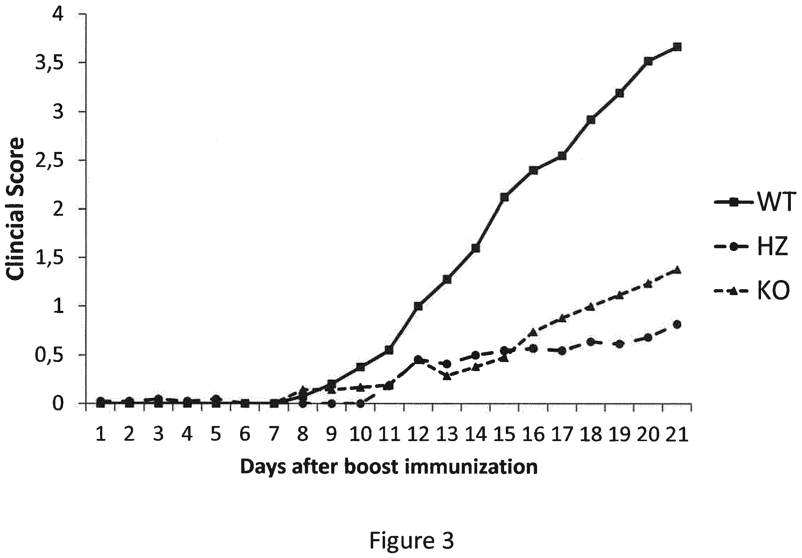

**Figure 3****:** Clinical arthritis scores during development of Collagen induced arthritis in wild-type (WT), heterozyguous (HZ) and GDF15 null mice (KO). GDF15 deficient (KO and HZ) show reduced and postponed inflammatory symptoms.

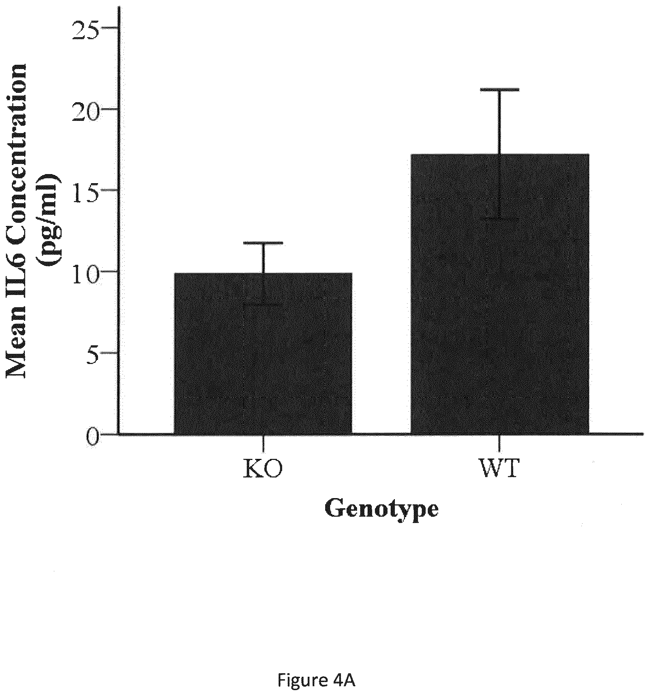

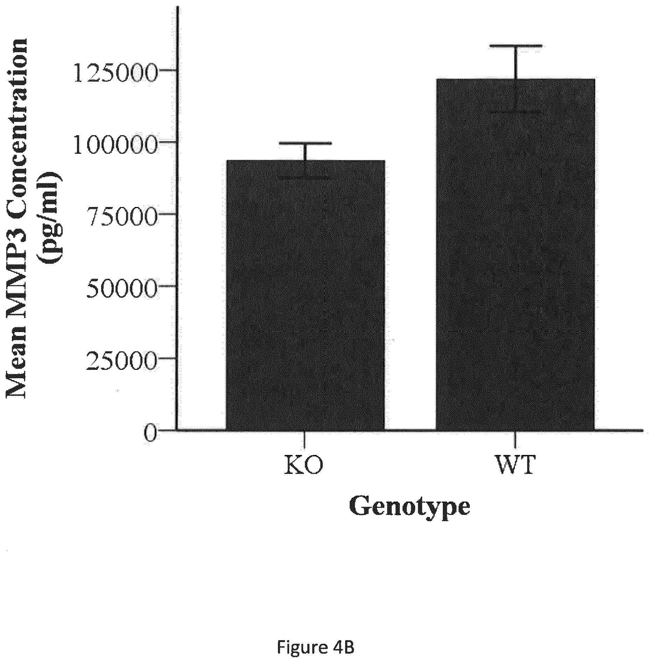

**Figure 4****:** Serum levels of IL-6 (**A**) and MMP3 (**B**), both known mediators of joint inflammation and destruction, in GDF15 null mice (KO) en wild-type littermate controls (WT). KO-mice show reduced levels of both inflammatory markers in the serum (p<0,05), further confirming reduced joint inflammation and destruction in GDF15 null mice.

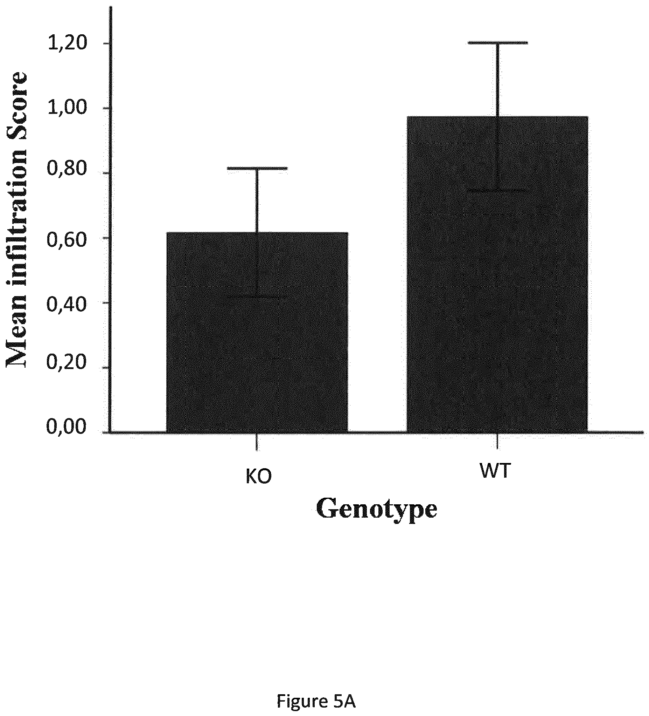

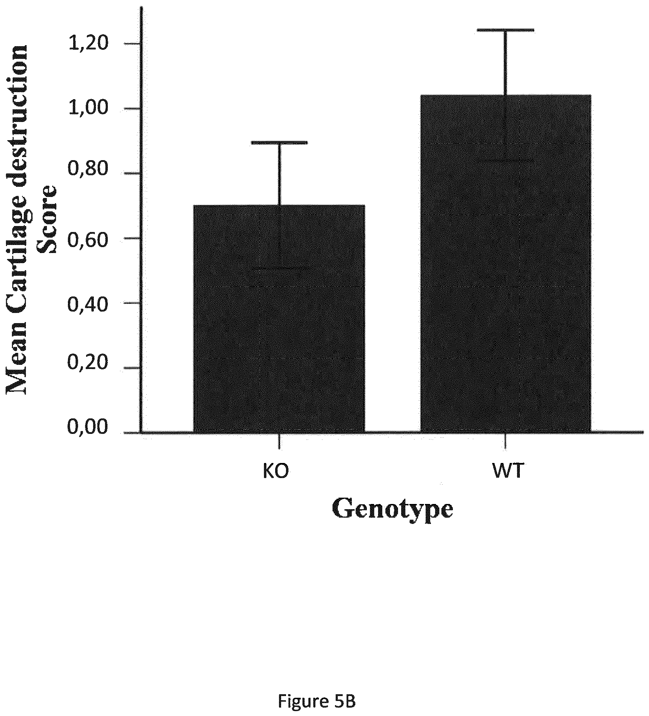

**Figure 5****:** Histological sections of KO and WT mice were prepared at the end of the CIA-experiment. Sections were scored for synovial infiltration and cartilage destruction by three independent, blinded observers. KO show reduced synovial infiltration (**A**) as well as cartilage destruction (**B**) further confirming the suppressed inflammation in GDF15 deficient mice.

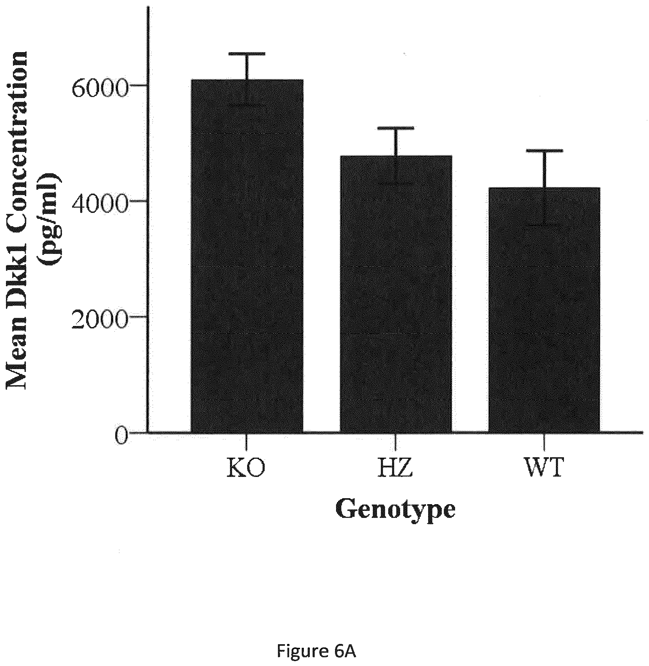

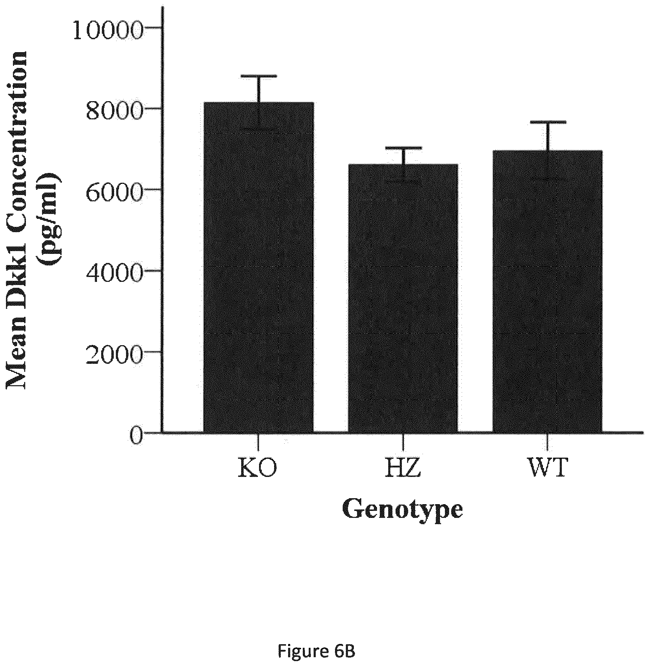

**Figure 6****:** Serum levels of Dkk1, a known mediator of bone metabolism under inflammatory conditions, were analyzed in serum from aged female (**A**) and male (**B**) mice (12 months). Reduced levels were detected in WT mice compared to GDF15 null mice, further confirming the involvement of GDF15 in joint metabolism.

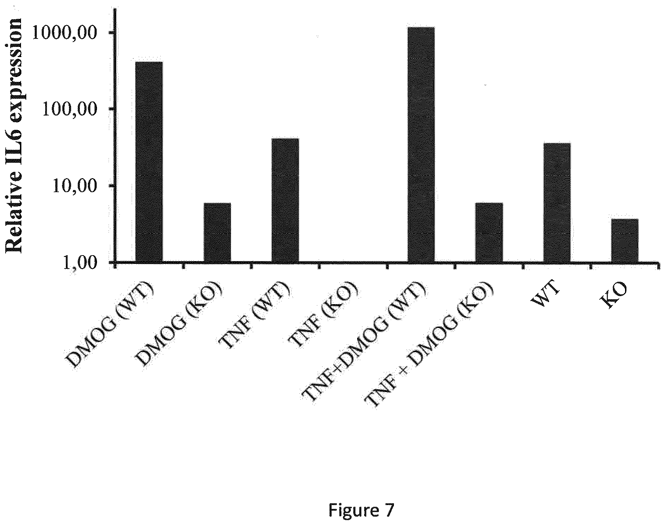

**Figure 7****:** IL6 expression is induced in wild-type synovial fibroblasts (WT) compared to GDF15 knock-out synovial fibroblasts (KO). This induction is even more pronounced in pro-inflammatory conditions induced by TNFalpha (10 ng/ml) (TNF) or hypoxic conditions induced by DMOG.

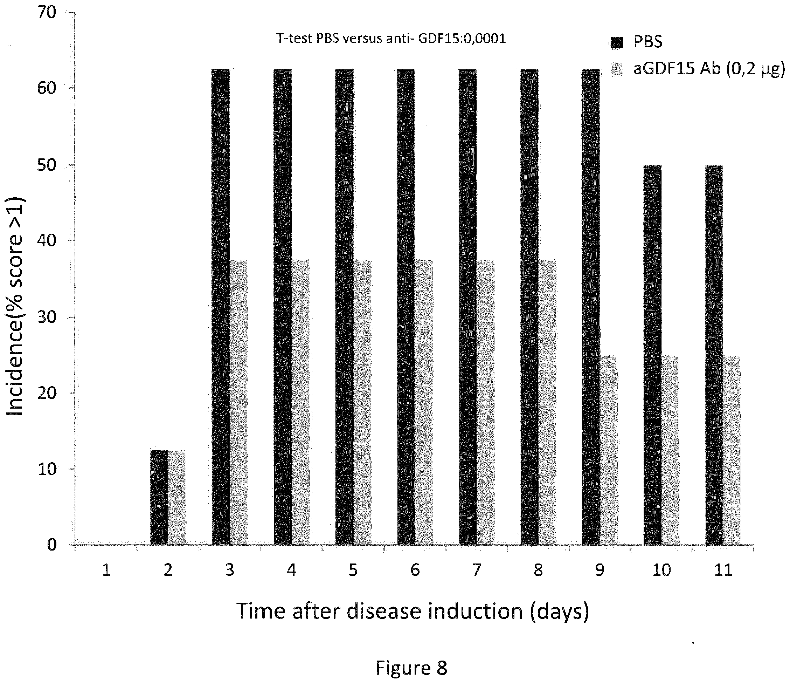

**Figure 8****:** Disease incidence in control (PBS) and treated (anti-GDF15 antibody) Collagen antibody induced arthritis (CAIA) mice, defined as any animal showing a clinical score ≥1 in at least one paw. Paired samples T-test PBS versus anti-GDF15: 0,0001.

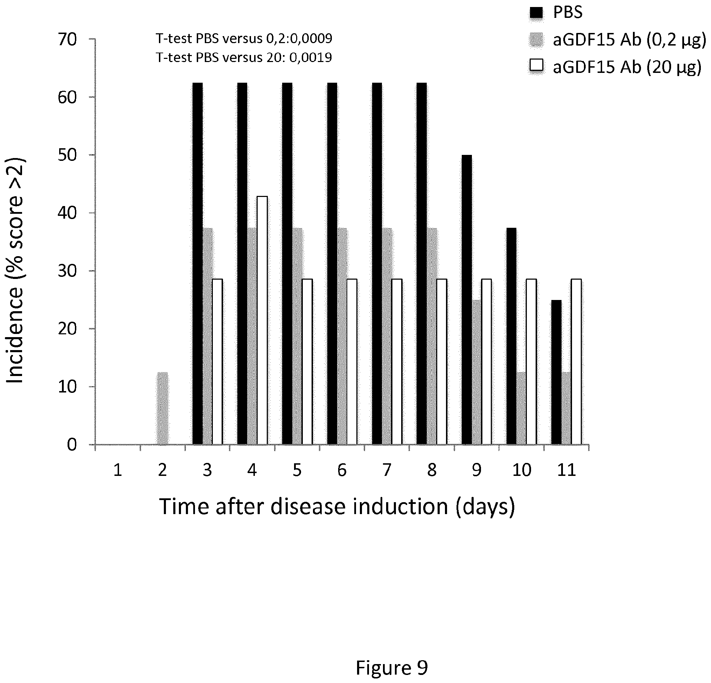

**Figure 9****:** Disease incidence in control (PBS) and treated (anti-GDF15 antibody) Collagen antibody induced arthritis (CAIA) mice, defined as any animal showing a clinical score ≥2 in at least one paw. Paired samples T-test PBS versus anti-GDF15: 0,0001

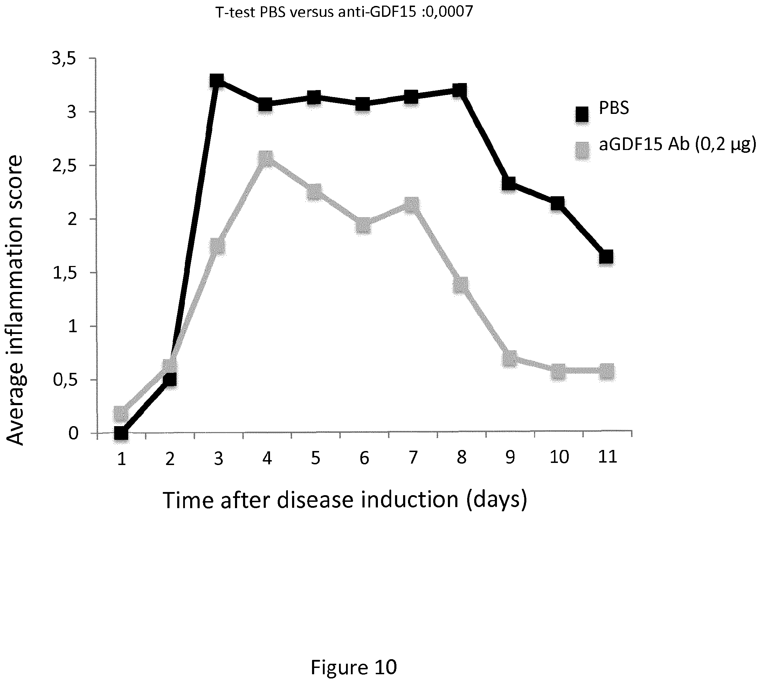

**Figure 10****:** Average clinical score values during development of Collagen antibody induced arthritis (CAIA) in PBS treated and anti-GDF15 antibody treated mice. Paired samples T-test PBS versus anti-GDF15: 0,0007.

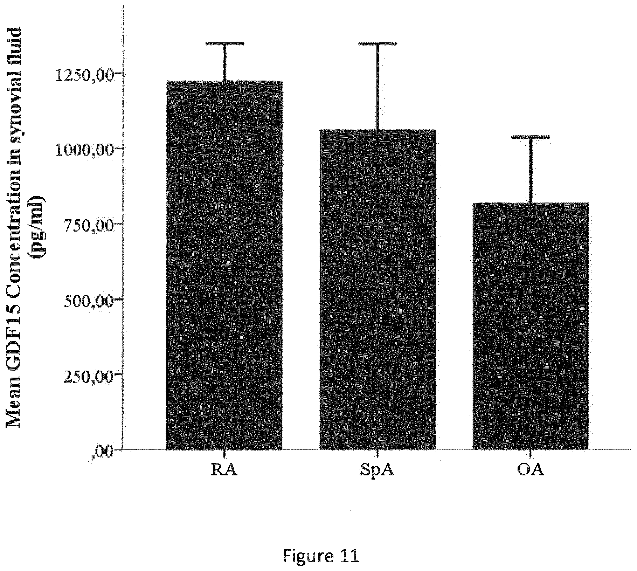

**Figure 11****:** Synovial fluid levels in RA (N23), SpA (19) and OA (6) corrected for age (patient age >55 years).

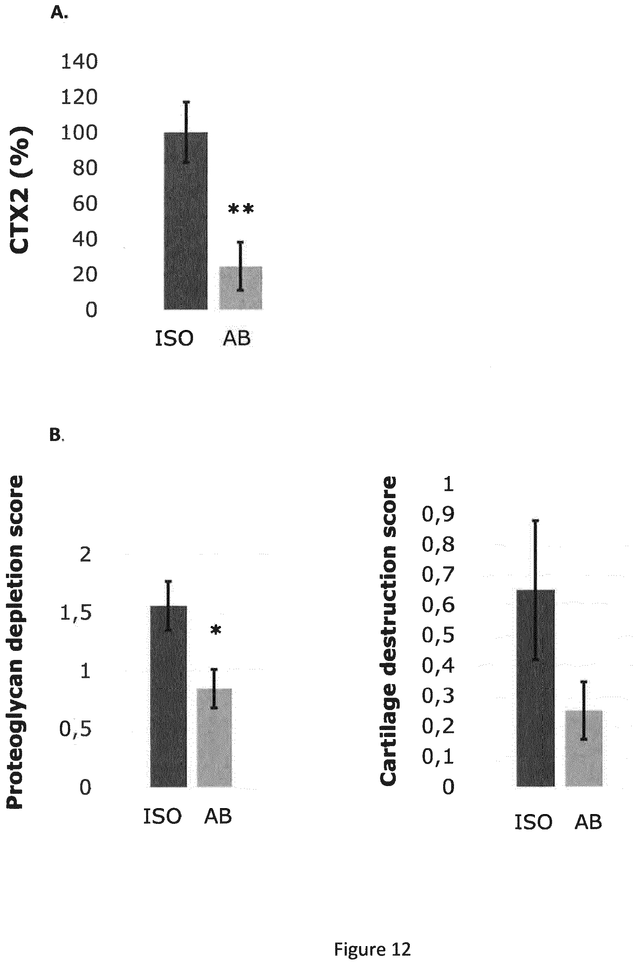

**Figure 12****:** Anti-GDF15 treatment impedes cartilage degradation in collagen antibody induced arthritis. (**A**) CTX-II serum levels, as a marker for cartilage degradation at day 16 after induction of arthritis with an anti-collagen antibody cocktail. Average CTX-II level in antibody treated mice (n=10) is shown in percentage as compared to the average level of CTX-II in the group of control mice (n=6). Error bars represent standard error of the mean. Significance of the lower level of CTX-II in the antibody treated mice was tested with Student's t-test with ** indicating p<0,01. (**B**) Histological scores of knees from isotype (n=9) and anti-GDF15 treated mice (n=16) 30 days after induction of CAIA. Particularly the extent of proteoglycan depletion in the cartilage was different in antibody treated mice compared to the control mice with Mann Whitney U tests with * indicating p<0,05.

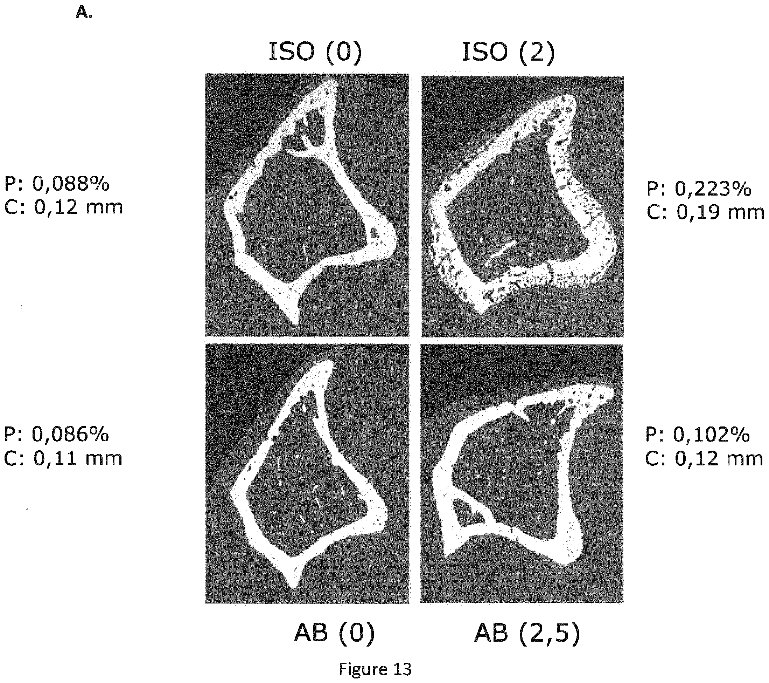

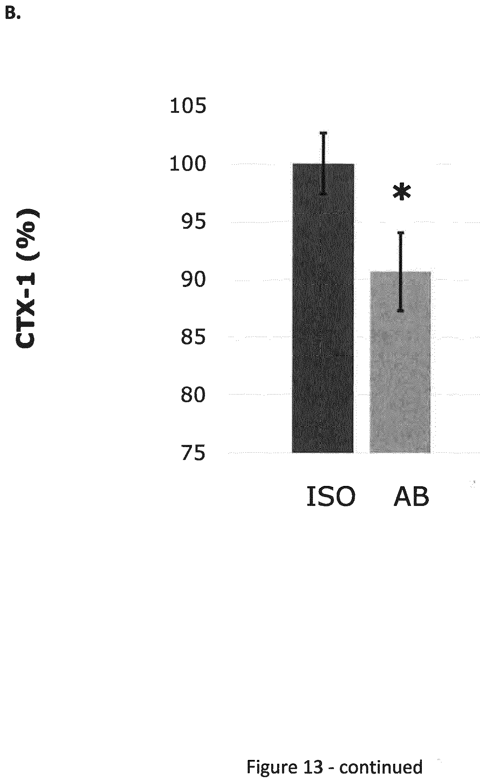

**Figure 13****:** Anti-GDF15 treatment decreases bone remodeling caused by collagen antibody induced arthritis. (**A**) Micro Computed Tomography slices of tibia showing a high level of porosity (bone resorption) and a compensating thickening of the cortical bone in a control mouse with severe arthritis at day 14. Cortical porosity and cortical thickness as measured and visualized in 3D by Fiji were obviously less pronounced in an anti-GDF15 treated mouse with a comparable clinical score of its hindleg (**B**) CTX-I serum levels, as a marker for bone destruction at day 16 after induction of severe arthritis (clinical score of > 5) with an anti-collagen antibody cocktail. Average CTX-I level in antibody treated mice (n=8) is shown in percentage as compared to the average level of CTX-I in the group of control mice (n=9). Error bars represent standard error of the mean. Significance of the lower level of CTX-I in the antibody treated mice was tested with Student's t-test with * indicating p<0,05.

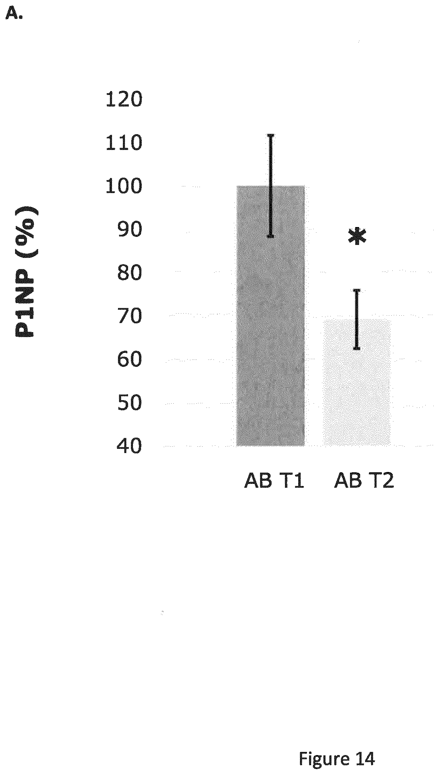

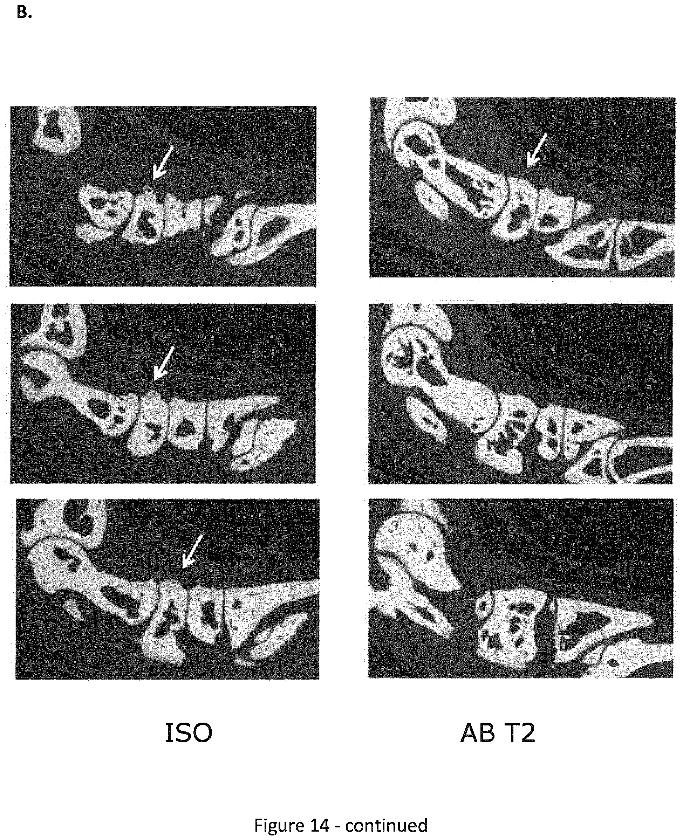

**Figure 14****:** Anti-GDF15 treatment diminishes bone new formation and remodeling after collagen antibody induced arthritis. (**A**) P1NP serum levels, as a marker for bone new formation at day 16 after induction of arthritis with an anti-collagen antibody cocktail. Average P1NP level of mice with anti-GDF15 treatment in the remodeling phase after arthritis from day 13 on (n=10) is shown in percentage as compared to the average level of P1NP in the group of mice treated during the inflammatory phase of arthritis from day 0-13 (n=10). Error bars represent standard error of the mean. Significance of the lower level of P1NP in the mice treated during remodeling was tested with Student's t-test with * indicating p<0,05. (**B**) Micro Computed Tomography slices of the foot of mice showing bone new formation (indicated by the white arrow) 30 days after arthritis induction in an isotype treated mouse. Anti-GDF15 treatment decreases bone new formation at the same area (white arrow) in a foot with comparable clinical inflammation score. Three slices per foot are shown.

### SUMMARY OF THE INVENTION

The present disclosure provides the GDF15 protein or gene as a therapeutic target for reducing inflammatory signs, disease severity and joint destruction.

A first embodiment relates to a Growth differentiation Factor 15 (GDF15) binding antibody, including GDF15 binding antibody fragments, decreasing the amount of GDF15 and/or decreasing the biological activity of GDF15, for use in treating rheumatoid arthritis, spondyloarthritides (ankylosing spondylitis (AS), SpA associated with psoriasis (psoriatic arthritis) and osteoarthritis (OA).

In a specific embodiment, the anti-GDF15 antibody is a monoclonal antibody, even more in particular a humanized monoclonal antibody.

In a further embodiment, the invention relates to a pharmaceutical composition comprising a GDF15 antibody decreasing the amount of GDF15 and/or decreasing the biological activity of GDF15, and a pharmaceutically acceptable excipient for use in treating rheumatoid arthritis (RA), spondyloarthritis (SpA) or psoriatic arthritis, or osteoarthritis.

### DETAILED DESCRIPTION OF THE INVENTION

The present invention will now be further described. In the following passages, different aspects of the invention are defined in more detail. As used in the specification and the appended claims, the singular forms "a", "an", and "the" include plural referents unless the context clearly dictates otherwise. By way of example, "a compound" means one compound or more than one compound. The terms described above and others used in the specification are well understood to those in the art.

It was demonstrated in the present invention that GDF15, a protein secreted by the connective tissue, is an active mediator of joint inflammation and contributes to inflammatory and remodeling features in arthritic joint diseases. The aim of the disclosure is to provide GDF15 as a therapeutic target in order to reduce inflammatory signs, disease severity and to modulate irreversible structural joint degeneration or bone spur formations.

A first aspect of the application relates to a Growth differentiation Factor 15 (GDF15) binding antibody for use in treating joint inflammation and/or joint remodeling, in particular joint destruction, in a subject, more in particular for use in treating an inflammatory joint disease, and even more particular for use in treating rheumatoid arthritis (RA), spondyloarthritis (SpA), psoriatic arthritis, or osteoarthritis.

"Joint inflammation" is characterised by pain, swelling, redness, stiffness and/or decrease in mobility of the joint, and can result from infection, trauma, degenerative changes, metabolic disturbances, autoimmune reaction, or other causes.

"Remodeling of joints" or "joint remodeling" is a key feature of inflammatory and degenerative joint disease. Bone erosion, cartilage degeneration and growth of bony spurs termed osteophytes are key features of structural joint pathology in the course of arthritis, which lead to impairment of joint function. Spondyloarthritides (SpA) in particular are characterised by the development of enthesitis and new bone formations (bony spurs, often termed syndesmophytes or enthesiophytes), evolving into ankylosis.

"Joint destruction" results from degradation and resorption of cartilage and bone in the joint. Bone resorption and apposition is a balance controlled by two distinct cell types, osteoclasts and osteoblasts, respectively. These cells are in closed relation by cell contacts, cytokines, growth factors and hormones. Interleukine (IL)-6 and Receptor of Activator of NF-[kappa]B Ligand (RANK-L) have been identified as the major cytokines implicated in osteoblast/osteoclast communications. Cartilage degradation is a consequence of an imbalance between anabolic and catabolic processes in the cartilage. This imbalance may be caused by cytokines, hormones and growth factors, such as IL1b, IL6, TNFalpha. Joint destruction is generally measured by traditional histological and CT-based techniques. It can further be detected clinically, radiographically, by MRI or by ultrasound. Cartilage damage is a key feature of degenerative joint disorders, primarily osteoarthritis (OA), and chronic inflammatory joint diseases, such as rheumatoid arthritis (RA). Osteoarthritis is a common disease that can cause severe pain and dysfunction in any joint, including the temporomandibular joint. The pathology is characterized by progressive cartilage degradation, subchondral bone remodeling, and chronic inflammation in the synovial tissue.

"Inflammatory joint disease" is a group of diseases that are referred to medically as arthridities (types of arthritis). The term " arthritis" is used medically to generally describe-diseases of the joints. The term, however, is also used to describe certain medical conditions, of which rheumatoid arthritis (RA) and spondyloarthritis (SpA) (including psoriatric arthritis) are the primary example, that consist of a multiplicity of different pathologic manifestations, including, but by no means limited to, joint disease. Discussions of arthritis may thus include diseases such as RA and SpA, where joint disorders are only one facet of the varied pathologies associated with the disease. For clinical purposes, two forms related to the predominant clinical manifestation - axial and peripheral SpA - and five subgroups- ankylosing spondylitis (AS), SpA associated with psoriasis and inflammatory bowel disease (IBD), reactive arthritis and undifferentiated SpA - are differentiated. The most important clinical features of the spondyloarthritides (SpA) are articular inflammation, erosion and new bone formation at peripheral and axial sites. Inflammation of attachment sites of ligaments and tendons to bones, enthesitis, is a hallmark of SpA which distinguishes it from other inflammatory rheumatic disorders. In addition, SpA is also characterised by new bone formation evolving into ankylosis, or into the formation of enthesophytes that also appear to originate from these insertion sites. Radiographic progression of disease reflecting structural damage is characterised by new bone formation leading to sacroiliac and spinal ankylosis. Both inflammation and progressive structural damage contribute to the burden of disease.

In one aspect, the specification is directed specifically to the joint disorder aspects of these diseases. The methods in the specification, however, may also have beneficial effects on non-joint-associated pathologies. For example, use of the methods of the specification to treat established joint inflammation associated with RA, osteoarthritis (OA), psoriatic arthritis, may also provide therapeutic benefits impacting on some of the other pathologic manifestations of these multifaceted disease states, such as vascular inflammation and nephritis. All of these disorders are characterized by elevated levels of GDF15 in the serum or locally in the joint (synovial fluid) (see figure 1, 2 and 11). Whereas most rheumatic diseases show elevated serum levels of GDF15 (figure 1), SpA (including psoriatic arthritis patients) and osteoarthritis patients show elevated levels of GDF15 in the synovial fluid (figure 2 and 11) and show high associations between disease activity (table 1) and synovial fluid levels of GDF15, pointing to a role for GDF15 in joint pathogenesis.

As used herein, "treating" or "treatment" is an approach for obtaining beneficial or desired clinical results. For purposes of this application, beneficial or desired clinical results include, but are not limited to, one or more of the following: lessening severity, alleviation of one or more symptoms such as inflammation, joint swelling, joint pain, and reduced mobility.

A "GDF15 binding antibody" binds specifically to GDF15. The term "binds specifically" as used herein is intended to indicate that a GDF15 binding moiety interacts preferentially with GDF15, and more specific with the secreted fragment of GDF15, and does not significantly interact with other proteins or other molecules. The binding of an antibody to GDF15 can be determined by standard methods, e.g. by ELISA, Western blotting, Label-free surface plasmon resonance, etc.

GDF15 binding moieties can include a variety of different types of molecules including those that specifically bind GDF15. Such GDF15 ligands include small molecules, polypeptides or nucleic acids (aptamers), antibodies and the like. Only GDF15 antibodies, including GDF15 binding antibody fragments, for use according to the claims belong to the invention.

In one aspect, a GDF15 binding moiety is an antibody. The term "antibody" refers to polyclonal antibodies, monoclonal antibodies (mAbs), chimeric antibodies, humanized antibodies, a human engineered antibody, a human antibody, as well as antigen binding antibody fragments and molecules having antigen binding functionality. More in particular, the term "antibody" includes an intact immunoglobulin having four polypeptide chains, two heavy (H) chains and two light (L) chains linked by disulfide bonds. The term "antibody" also includes GDF15 binding antibody fragments illustratively including, but not limited to, such fragments as a Fab fragment, a Fab' fragment, a F(ab')2 fragment, a Fd fragment, a Fv fragment, a scFv fragment, heavy chain antibodies (hcAb), minibodies [30], a variable domain of camelid heavy chain antibody (VHH or Nanobody®), a variable domain of the new antigen receptor (VNAR) and engineered CH2 domains (nanoantibodies; [27]). Active fragments can be derived from an antibody of the present invention by a number of art-known techniques. For example, purified monoclonal antibodies can be cleaved with an enzyme, such as pepsin, and subjected to HPLC gel filtration. The appropriate fraction containing Fab fragments can then be collected and concentrated by membrane filtration and the like. For further description of general techniques for the isolation of active fragments of antibodies, see for example, Khaw et al. and Rousseaux et al.

An anti-GDF15 antibody and/or GDF15 binding antibody fragment is capable of specifically binding GDF15. Anti-GDF15 antibodies may be provided by any method, illustratively including, but not limited to, immunization, isolation and purification, enzymatic cleavage of an intact immunoglobulin, screening of phage display libraries, chemical synthesis of a desired GDF15 binding peptide or protein, production by recombinant nucleic acid technology. Combinations of such methods may also be used. Anti-GDF15 antibodies are commercially available and described e.g. in WO2001/81928. In a specific aspect, the specification is directed to the monoclonal antibody, including an antigen binding fragment thereof, which is produced by hybridoma cell line 52D4C1, deposited in the Belgian Coordinated Collections of Micro-organisms (BCCM) - LMBP Plasmid Collection - Ghent University -Technologiepark 927 - 9052 Gent - Belgium, on 30 September, 2014, and given deposit number LMBP 10815CB for use to treat diseases as described herein. In an even more specific embodiment, said monoclonal antibody for use as described herein is a humanized monoclonal antibody.

Competition for binding can be evaluated for any pair of antibodies (including fragments thereof). For example, using the appropriate detection reagents, the binding specificity of antibodies or binding fragments from any source can be compared to the binding specificity of the monoclonal antibodies disclosed herein. The antibodies may be assayed for specific binding by any method known in the art. Many different competitive binding assay format(s) can be used. The immunoassays which can be used include, but are not limited to, competitive assay systems using techniques such western blots, radioimmunoassays, ELISA, "sandwich" immunoassays, and immunoprecipitation assays. Such assays are routine and well known in the art (see, e.g., Ausubel et al., eds, 1994 Current Protocols in Molecular Biology, Vol. 1, John Wiley & sons, Inc., New York). Additionally, routine cross-blocking assays such as those described in Antibodies, A Laboratory Manual, Cold Spring Harbor Laboratory, Ed Harlow and David Lane, 1988, can be performed.

An anti-GDF15 antibody can be made by immunization using as an antigen a full length GDF15 or a peptide fragment of GDF15, in particular the mature protein. "Growth differentiation factor 15" (GDF15) is a protein belonging to the transforming growth factor beta superfamily that has a role in regulating inflammatory and apoptotic pathways in injured tissues and during disease processes. GDF15 is also known as Macrophage-inhibiting Cytokine 1(MIC-1), Prostate Derived Factor (PDF), and Placental Bone Morphogenetic Protein (PLAB). The premature protein consists of 308 amino acids that contain a signal peptide, a propeptide, and a mature protein. The mature protein is secreted as a homodimer linked by disulfide bonds and arises by cleavage of the dimeric precursor at a conserved RXXR site that can be found at either amino acid 193 or 196 [1]. The mature protein contains two additional cysteine residues in addition to the seven conserved cysteines necessary for the cysteine knot, a structural hallmark of this TGF-β superfamily. Such proteins and peptides may be, illustratively a human, pig, sheep, rat, mouse, monkey, ape, or other GDF15 protein or peptide. Human GDF15 protein and nucleic acid sequences included herein are any homolog or artificial sequence that is substantially identical, i.e. at least 75%, 80%, 85%, 87%, 89%, 90%, 91%, 92%, 93%, 94%, 95%, 96%, 97%, 98%, 99% or 100% identical to the corresponding GDF15 sequence identified by NCBI Accession number NP_004855.2 GI:153792495 and NC_000019.9 GI:224589810, in particular to the mature chain identified in said sequence. Amino acid sequences for GDF-15 are also disclosed in [1] and WO99/06445.

Antigens may be prepared by any of various methods, including isolation from natural sources, recombinant production or by chemical synthetic techniques. GDF15 proteins and peptides for use as antigens in preparation of a GDF15 binding antibody are similarly prepared by any of various techniques.

A peptide portion of GDF15 or other antigen may be made more immunogenic if desired by linkage to a carrier molecule such as bovine serum albumin or keyhole limpet hemocyanin. Such a linkage may be accomplished by any of various techniques, illustratively including, but not limited to, conjugation and expression of a fusion protein.

Antibodies, antigen binding fragments and methods for their generation are known in the art and such antibodies, antigen binding fragments and methods are described in further detail, for instance, in Antibody Engineering, Kontermann, R. and Dubel, S. (Eds.), Springer, 2001; Harlow, E. and Lane, D., Antibodies: A Laboratory Manual, Cold Spring Harbor Laboratory Press, 1988; Ausubel, F. et al., (Eds.).

As an alternative aspect of the specification, other compounds or methods decreasing the amount of GDF15 in a subject or decreasing the biological activity of GDF15 in a subject can be used. Hence, in a further aspect the application relates to a compound decreasing the amount of GDF15 and/or decreasing the biological activity of GDF15 in a subject for use in treating joint inflammation and/or joint remodeling, in particular joint destruction, in a subject, more in particular for treating an inflammatory joint disease, and even more particular for treating one or more of the diseases selected from the group consisting of rheumatoid arthritis (RA), spondyloarthritis (SpA) (including psoriatric arthritis (PsA)), and osteoarthritis (OA). Examples of said compounds include catalytic and inhibitory oligonucleotide molecules targeted against the GDF15 gene (eg ribozymes, DNAzymes, antisense RNA, and small inhibitory RNA (siRNA)), and inhibitors of GDF15 transcription or translation. Alternatively, the GDF15 inhibiting agent may inhibit the activity of endogenous GDF15 in the subject, and may be selected from soluble extra-cytoplasmic receptor domains of GDF15 receptors, other soluble molecules or matrix-associated proteins that bind to GDF15 (e.g. heparin, heparan sulphate and fetuin), and peptide, peptide mimetic, or small organic molecule inhibitors of, for example, GDF15 binding to its receptor. Additionally, peptide, peptide mimetic, or small organic molecule inhibitors might inhibit the activity of endogenous GDF15 by inhibiting GDF15 receptor phosphorylation, or transmission of signaling information from the GDF15 receptor to the cell nucleus, or action of the relevant transcription factor(s) on the cell genome. Further, the GDF15-inhibiting agent may be an inhibitor of the proconvertase enzyme responsible for cleaving the propeptide from the mature GDF15 protein domain. Proconvertase enzyme may be inhibited by, for example, (a) transfection of cells with an alpha-1-antitrypsin mutant, alpha-1-antitrypsin Portland, (b) polyarginine peptides; and (c) peptides based on the sequence of the target protein for the proconvertase, spanning the propeptide sequence and proconvertase sequence of the target protein. Only GDF15 antibodies, including GDF15 binding antibody fragments, for use according to the claims belong to the invention.

The method of the present specification is useful for the treatment of a subject suffering from joint inflammation. More specific, the subject is suffering from an inflammatory joint disease, especially rheumatoid arthritis (RA) or spondyloarthritis (SpA). In some aspects, the specification provides methods for treating and/or alleviating clinical symptoms of rheumatoid arthritis (RA), spondyloarthritis (SpA), psoriatric arthritis or osteoarthritis in a subject.

In another aspect, the specification provides antibodies binding GDF15 for use to reduce incidence of, ameliorate, suppress, palliate, and/or delaying the severity, the development or the progression of an (inflammatory) joint disease in an individual, in particular diseases characterized by joint inflammation, joint remodeling (e.g. cartilage degeneration or formation of bony spurs) and/or joint destruction. Exemplary diseases include rheumatoid arthritis, spondyloarthritides (ankylosing spondylitis (AS), SpA associated with psoriasis (psoriatic arthritis), reactive arthritis and undifferentiated SpA), osteoarthritis (OA), and juvenile onset rheumatoid arthritis.

An "individual" or "subject" is a vertebrate, preferably a mammal, more preferably a human. Mammals include, but are not limited to pets (such as cats, dogs and horses), primates, mice and rats.

In a further aspect, the specification includes a pharmaceutical composition comprising an GDF15 binding antibodydecreasing the amount of GDF15 and/or decreasing the biological activity of GDF15, and at least one pharmaceutically acceptable carrier, diluent or excipient and/or adjuvant, and optionally one or more further pharmaceutically active compounds. Pharmaceutically acceptable excipients are known in the art, and are relatively inert substances that facilitate administration of a pharmacologically effective substance. For example, an excipient can give form or consistency, or act as a diluent. Suitable excipients include but are not limited to stabilizing agents, wetting and emulsifying agents, salts for varying osmolarity, encapsulating agents, buffers, and skin penetration enhancers. Excipients as well as formulations for parenteral and nonparenteral drug delivery are set forth in Remington, The Science and Practice of Pharmacy 20th Ed. Mack Publishing (2000). Further suitable carriers and diluents are for example lactose, dextrose, sucrose, sorbitol, mannitol, starches, gum acacia, calcium phosphate, alginates, tragacanth, gelatin, calcium silicate, microcrystalline cellulose, polyvinylpyrrolidone, polyethylene glycol, cellulose, (sterile) water, methylcellulose, methyl- and propylhydroxybenzoates, talc, magnesium stearate, edible oils, vegetable oils and mineral oils or suitable mixtures thereof. In addition, co-solvents such as alcohols may improve the solubility and/or the stability of the compounds.

The GDF15 binding antibody decreasing the biological activity of GDF15, as described herein may be administered alone, or in combination with other active ingredients that improve the therapeutic effect, whether administered in combination, serially or simultaneously. For example, the pharmaceutical composition may further comprise a therapeutically effective amount of at least one TNF-alpha inhibitor, NSAID, steroid, or disease modifying anti-rheumatic drug (e.g. MTX).

The GDF15 binding antibody can be administered to an individual via any suitable route. Accordingly, in some aspects, the GDF15 binding antibody is administered to an individual in accord with known methods, such as intravenous administration, e.g., as a bolus or by continuous infusion over a period of time, by intramuscular, intraperitoneal, intracerebrospinal, subcutaneous, intra-articular, sublingually, intrasynovial, via insufflation, intrathecal, oral, inhalation or topical routes. Administration can be systemic, e.g., intravenous administration, or localized. Suitable solvents are, for example, water, physiological saline solution or alcohols, e.g. ethanol, propanol, glycerol, in addition also sugar solutions such as glucose or mannitol solutions, or alternatively mixtures of the various solvents mentioned. The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

Commercially available nebulizers for liquid formulations, including jet nebulizers and ultrasonic nebulizers are useful for administration. Liquid formulations can be directly nebulized and lyophilized powder can be nebulized after reconstitution. Alternatively, a GDF15 binding antibody can be aerosolized using a fluorocarbon formulation and a metered dose inhaler, or inhaled as a lyophilized and milled powder.

In one aspect, a GDF15 binding antibody is administered via site-specific or targeted local delivery techniques. Examples of site-specific or targeted local delivery techniques include various implantable depot sources of the GDF15 binding antibody or local delivery catheters, such as infusion catheters, an indwelling catheter, or a needle catheter, synthetic grafts, adventitial wraps, shunts and stents or other implantable devices, site specific carriers, direct injection, or direct application.

Various formulations of an GDF15 binding antibody may be used for administration. In some aspects, the GDF15 binding antibody may be administered neat. In some aspects, the GDF15 binding antibody and a pharmaceutically acceptable excipient may be in various formulations.

The GDF15 binding antibody will generally be administered in an "effective amount", by which is meant any amount that, upon suitable administration, is sufficient to achieve the desired therapeutic effect such as reduction of joint inflammation, destruction and joint pain in the individual to which it is administered.

Usually, depending on the condition to be prevented or treated and the route of administration, such an effective amount will usually be between 1µg and 30mg per kilogram body weight of the patient per day, more often between 0,5mg and 10mg per kilogram body weight per day of the patient per day, which may be administered as a single daily dose, divided over one or more daily doses, or essentially continuously, e.g. using a drip infusion, or as a weekly or monthly dose. The amount(s) to be administered, the route of administration and the further treatment regimen may be determined by the treating clinician, depending on factors such as the age, gender and general condition of the patient and the nature and severity of the disease/symptoms to be treated. Reference is made to the standard handbooks, such as the latest edition of Remington's Pharmaceutical Sciences. Similarly suitable administration forms - which may be solid, semi-solid or liquid, depending on the manner of administration - as well as methods and carriers, diluents and excipients for use in the preparation thereof, will be clear to the skilled person.

The invention will now be illustrated by means of the following examples, which do not limit the scope of the invention in any way.

### EXAMPLES

### 1. Materials and Methods

### 1.1 Patients

The studied samples were derived from different populations.

The first cohort consisted of 1024 patients with rheumatic symptoms, from whom serum samples were consecutively sent to our laboratory for ACPA determination within the context of a diagnostic investigation [10]. Patients were diagnosed by a clinician by reviewing of files. Eighty-one patients were lost to follow up. We thus diagnosed 92 individuals as having RA, and all of these patients met the ACR criteria for RA [11]. In 463 patients the diagnosis of RA could be excluded. In these non-RA patients, the most frequent diseases were degenerative joint diseases, spondyloarthropathy, systemic lupus erythematosus, vasculitis , polymyalgia rheumatic, crystalin induced arthropathies, Sjögren's disease and fibromyalgia. A further 388 patients had undifferentiated arthritis and were further withdrawn from the analysis. The details of this cohort have been described elsewhere [10]. Rare diagnoses (i.e. 5 cases or less) were not included for analysis.

We next aimed to confirm the results in a second study group and to compare serum and synovial fluid samples. This cohort included samples from consecutive patients with an indication for an arthroscopic procedure for diagnostic purposes. From this population patients with well-established clinical diagnosis were selected: 37 patients meeting ACR RA-criteria [11], 63 patients meeting the ESSG SpA-criteria [12] and 17 OA patients. Synovial fluid was obtained from RA and SpA patients. In a selected number of patients, synovial tissue sample were used for qPCR.

These studies have been approved by the local ethical committee and all patients gave informed consent.

### 1.2 Analysis of GDF15 levels

Serum samples and synovial fluid samples were aliquoted and stored at -80 °C until analysis. The levels of GDF15 were analyzed using the GDF15 DuoSet ELISA (R&D systems, Abingdon, UK), according to the manufacturer's instructions. The inter-assay precision is determined at 14,8 (%CV) and was assessed by repeated measures of an in-house control sample in 12 consecutive assays. Each sample was assayed in duplicate.

### 1.3 Animal Experiments

To dissect the role of GDF15 in distorted joint homeostasis during arthritic diseases in vivo, we apply anti-GDF15 antibodies in different arthritis models each with its own characteristics. Anti-GDF15 antibodies were obtained using standard procedures by immunizing Lou/C rats with purified (>95%) recombinant mouse GDF15. The secreted fragment of Recombinant mouse GDF15 (AHHHHHHPGGPGSDELDSAHAHPRDSCPLGPGRCCHLETVQATLEDLGWSDWVLSPRQLQLSM CVGECPHLYRSANTHAQIKARLHGLQPDKVPAPCCVPSSYTPVVLMHRTDSGVSLQTYDDLVARG CHCA; SEQ ID NO: 1) was produced in HEK293T cells. Selected clones were fused with the myeloma cell line P3-X63-Ag8. The resulting hybridoma's were screened by ELISA. Positive clones were expanded and subcloned. The specificity of the Ab was demonstrated by ELISA and WB. Biological activity was demonstrated by inhibition of in vitro osteoclast assays. The monoclonal antibody produced by hybridoma cell line 52D4C1 was deposited in the Belgian Coordinated Collections of Micro-organisms (BCCM) - LMBP Plasmid Collection - on 30 September, 2014, and given deposit number LMBP 10815CB. The hybridoma for the isotype control was purchased at ATCC, USA, and treated similar to the rat anti-mSn Ab. Hybridomas were grown to confluency in DMEM 10% IgG depleted FCS, 1% P/S and medium was harvested up to six times. The Ab was purified from the medium on a protein G column to a purity of > 95%, protein containing fractions were pooled and dialysed to PBS with endotoxin levels < 1EU/ml.

Four different disease models are used to evaluate the effect of GDF15 deficiency using GDF15 knock-out mice and/or anti-GDF15 antibody treatment. First, the antibody is given to TNF overexpressing models. This model is primarily characterized by joint inflammation and destruction and does not show signs of remodeling (new bone formation) response. Second, anti-GDF15 antibodies are injected in the CIA-model and mice are analyzed at early and late stage. The former is primarily characterized by inflammation induced destruction, the latter stage shows obvious signs of bone new formation. Third, aging DBA/1 mice, known as a very suitable model to study bone new formation, are injected with an anti-GDF15 antibody. Fourth, a CAIA mouse model is used to further analyze the role of GDF15 in joint destruction and bone new formation.

### In vivo evaluation in Collagen Induced Arthritis (CIA) using GDF15 knock-out mice.

GDF15 deficient mice were kindly provided by dr. Lee from John Hopkins University, Baltimore, USA [15]. Mice were bred from heterozyguous parents. All animal experiments were approved by the local ethics committee of Ghent University.

In a first experiment, KO, HZ and WT mice were sacrificed at 3 and 12 months of age. Hind paws were processed for histology and serum was collected. Serum of the mice was analyzed for Dkk1, using a commercial sandwich ELISA assay (R&D Systems) specific for mouse Dkk1, according to the manufacturer's protocol. In a second experiment inflammatory arthritis was induced by chicken collagen in the three genotypes, as described before [16] and modified for C57BI/6 mice by the use of 250 µg mycobacterium Tuberculosis per mouse in immunization and boost. In this experiment, mice were clinically observed and scored for swollen ankle joints. At sacrifice, hind paws were processed for histology and serum was collected.

In a third experiment, synovial tissue was isolated from hind paws of GDF15 null and wild-type mice. Synovial fibroblast were isolated from the tissue and cultured. To mimic inflammatory conditions, cells (passage 2-3) were stimulated with TNFa (10 ng/ml) and DMOG (a compound to mimic hypoxic conditions as seen in inflammation). After 24h, cells were isolated and gene expression of IL-6 was analyzed by qPCR.

### Histological evaluation of severity of bone damage in mice

Knees were fixed in 4% formaldehyde, decalcified and embedded in paraffin. Serial sections of the knee were stained with hematoxylin and eosin (H&E) or with saffranin O-fast green and inflammation and joint damage of the femorotibial and femoropatellar joints were investigated by scoring five parameters as follows: inflammation was scored on a scale of 0 (no inflammation) to 3 (severe inflamed joint) depending on the number of inflammatory cells in the synovial cavity (exudate) and synovial tissue (infiltrate). Exudate and inflammatory infiltrate were both assigned individual scores. Loss of proteoglycans was scored on a scale of 0 to 3, ranging from fully stained cartilage to destained cartilage or complete loss of articular cartilage. Cartilage destruction was scored on a scale of 0 to 3, ranging from the appearance of dead chondrocytes (empty lacunae) to complete loss of the articular cartilage. Loss of bone was scored on a scale of 0 to 5 ranging from no damage to complete loss of the bone structure. A composite score was calculated by summing the individual parameters. Scoring was executed blindly by three investigators and mean values were calculated.

### In vivo evaluation in Collagen Antibody Induced Arthritis (CAIA) using anti-GDF15 antibody

A. Collagen antibody induced arthritis (CAIA) was induced into C57BL/6 mice (n=7) by passive transfer of anti-collagen II antibodies (Harlan Laboratories) at day 0 (120 µl total volume of purified mAb in PBS). At day 3, mice received an LPS boost (25 µg/mouse ip). Mice received daily administration of 0,2 µg anti-GDF15 mAbs or PBS from day-1 till day 13, after which they were sacrificed.
B. Mice were intravenously injected with 2,4 mg ArthritoMabTM antibody cocktail for C57BI/6 mice (Mdbiosciences). Three days later mice were intraperitoneally challenged with LPS. The mice were daily treated from the day of antibody cocktail administration on. One group of mice was treated after the inflammatory phase from day 13 on. Treatments consisted out of intraperitoneally injections with either isotype or the anti-GDF15 antibody in PBS. Mice were monitored daily for clinical symptoms of arthritis. Serum samples were collected on day 16. Mice were sacrificed on day 30. Clinical severity was graded as follows: 0 = normal; 0.5 = erythema and edema in only one digit; 1 = erythema and mild edema of the footpad, or ankle or two to five digits; 2 = erythema and moderate edema of two joints (footpad, ankle, two to five digits); 3 = erythema and severe edema of the entire paw; 4 = reduced swelling and deformation leading to incapacitated limb. The individual mouse arthritic score was obtained by summing the scores recorded for each limb. Clinical evaluations were performed by two investigators unaware of mouse identity and the mean of both scores was calculated. Bone degradation marker C-terminal telopeptide α1 chain of type I collagen (CTX-I), cartilage degradation marker CTX-II and bone new formation marker procollagen type 1 N-terminal propeptide P1NP were measured by ELISA according to the manufacturer's instructions (Immunodiagnosticsystems). Upon sacrifice, joints are evaluated histologically, using specific stainings (e.g. Safranin O, Hematoxylin/Eosin, TRAP) to estimate the impact on structural changes in joint tissues (cartilage, tendon, meniscus, synovium and bone). In parallel, imaging techniques (such as RX, µCT and MRI) are used to detect early changes in joint structures. These have the unique advantage of being able to be performed longitudinally over time. Moreover, biochemical parameters for cartilage and bone degradation and turnover (COMP, CTXI, CTXII, MMP3, IL6) are evaluated.

### Micro Computed Tomography

Optimal scanner settings were selected based on the sample size and composition. The samples were scanned on HECTOR, using a directional X-ray source set at 100kV, 1 mm Aluminum filtration, 10Watt beam power. The detector was a Perkin-Elmer flat panel measuring 40x40cm, with a pixel pitch of 200µm. The magnification was set at 40 times resulting in a voxel pitch inside the sample of 5µm. A total of 2000 projections of 1 second exposure time each was recorded. The data were then reconstructed using Octopus [34], a commercial software originally developed by UGCT which uses a custom implementation of the standard FDK-algorithm for reconstruction of cone-beam CT data. 3D visualisations and calculations were made using the commercial rendering software VGStudioMAX (Volume Graphics) or Fiji.

### 1.4 Statistical analysis

Multiple comparisons were done using One-Way ANOVA and Bonferroni's post hoc tests. Two-group comparisons were done using student's test, unless otherwise stated. In order to normalize the data, parametric tests were performed using log-transformed serum and synovial fluid concentrations. For clarity of interpretation, the figures report the non-transformed concentrations. Correlations were investigated by using Spearman's Rho correlation test.

### 2. Results

### 2.1 Low serum GDF15 levels in SpA as opposed to other inflammatory rheumatic diseases.

Initially, steady state GDF15 serum levels were measured in a variety of rheumatic diseases. Therefore, a cohort of 555 patients, visiting the outpatient clinic of the Rheumatology department of Ghent University Hospital, was screened. The mean concentration of GDF15 was significantly different between groups (p<0,001). In SpA patients the GDF15 serum levels were markedly lower compared to RA (p<0,001) and SLE (p=0,04) patients, while patients suffering from degenerative joint disease show an intermediate, though statistically significant different (p=0,004) level of GDF15 (figure 1). No significant differences were observed between subdiagnoses of SpA (psoriatic arthritis vs non-PsA SpA) (data not shown). Interestingly, elevated levels of GDF15 were also detected in other connective tissue diseases such as vasculitis pathologies (p<0,001), crystal induced arthropathies (p<001), and polymyalgia rheumatica (p<0,001). The observed differences in GDF15 serum levels between RA, SpA and SLE patients were further explored and confirmed in the second independent cohort (figure 2, panel A).

### 2.2 Elevated GDF15 synovial fluid versus serum levels particularly in PsA patients.

We next compared serum and synovial fluid levels in patients suffering from RA and SpA (non-PsA SpA and PsA) and related this to clinical characteristics. Interestingly, PsA patients show a significant higher concentration of GDF15 in paired samples of synovial fluid compared to serum. These differences were not observed in RA nor in non-PsA SpA-patients. For PsA as well as RA patients a significant correlation was observed between the GDF15 serum concentration and synovial fluid concentration (PsA: R=0,395; RA: R=0,503), whereas non-PsA SpA patients showed no correlation (see table 1). Importantly, after correction for age (> 55 year), synovial fluid levels were found to be higher than serum levels and are comparable in RA, SpA and OA (figure 11).

**Table 1: Spearman's Rho correlation coefficients for correlations between the GDF15 serum and synovial fluid concentrations and routine biochemical and clinical inflammation parameters for RA (n=37) and PsA (n=30) and non-PsA SpA (n=33) patients. For RA, patients values are shown for the second study (section 2.2) and the third study at baseline.**

| | | RA Patients | | PsA Patients | | Spa non PsA Patients | |
|---|---|---|---|---|---|---|---|
| | | Correl Coeff | Sign | Correl Coeff | Sign | Correl Coeff | Sign |
| GDF15 Serum | ESR | *0,555*/0,073 | *0,001*/ns | 0,261 | ns | -0,096 | ns |
| | CRP | 0,329/0,035 | ns/ns | 0,160 | ns | -0,051 | ns |
| | ACPA | 0,132/0,150 | ns/ns | 0,389 | ns | -0,323 | ns |
| | SJC | 0,105/0,085 | ns/ns | 0,109 | ns | 0,281 | ns |
| | MMP3 | 0,051 | ns | 0,327 | ns | -0,208 | ns |
| | MMP1 | 0,083 | ns | 0,131 | ns | 0,199 | ns |
| | MMP3(SF) | 0,005 | ns | 0,167 | ns | 0,017 | ns |
| | MMP1(SF) | -0,179 | ns | 0,104 | ns | 0,011 | ns |
| GDF15 Syn FI | ESR | 0,192 | ns | 0,166 | ns | *0,356* | *0,04* |
| | CRP | 0,187 | ns | 0,242 | ns | *0,568* | *<0,001* |
| | ACPA | -0,086 | ns | -0,062 | ns | 0,012 | Ns |
| | SJC | 0,207 | ns | *0,449* | *0,02* | 0,203 | Ns |
| | MMP3 | 0,050 | ns | *0,434* | *0,03* | *0,577* | *0,001* |
| | MMP1 | 0,072 | ns | -0,177 | ns | 0,118 | ns |
| | MMP3(SF) | 0,188 | ns | *0,465* | *0,03* | *0,541* | *0,004* |
| | MMP1(SF) | 0,150 | ns | *0,466* | *0,03* | *0,659* | *<0,001* |
| | GDF15(serum) | *0,503* | *0,002* | *0,395* | *0,04* | -0,009 | ns |

| | | | | | | | |
|---|---|---|---|---|---|---|---|
| Correl Coeff= Spearman's Rho correlation coefficient; Sign= indicates statistical significance, p<0,05. All variables are measured in serum, except when marked with "(SF)" (synovial fluid). GDF15 concentrations were also correlated to conventional biochemical and clinical parameters (CRP, ESR, SJC, ACPA, MMP3 and MMP1) (see table 1). Remarkably, SpA patients, showed a positive correlation between GDF15 synovial fluid levels and of MMP3 and MMP1. The MMP3-GDF15_{synovial fluid} correlation was most pronounced and highly significant in non-PsA SpA patients. In these patients, GDF15 synovial fluid levels also correlated with inflammation parameters (CRP, ESR) and MMP1 levels (see table 1). Given the involvement of MMPs in joint degradation, the observed correlations further confirm the relationship between synovial GDF15 levels and disease activity in SpA patients. | | | | | | | |

### 2.3 GDF15 knock-out mice show reduced inflammation in a collagen-induced arthritis (CIA) model.

Under steady-state conditions, no dramatic changes were observed in joint metabolism and architecture. There was a trend towards higher COMP levels in KO mice, though not statistically significant. Interestingly, DKK1 levels were elevated in knock-out mice compared to WT littermates (figure 6).

In a second set of animal experiments, mice were challenged to develop joint inflammation, using the Collagen Induced Arthritis (CIA) model. This model is the state-of-the-art model for joint inflammation in general. In the early phase of the model, joint inflammation is associated with joint destruction and shows typical RA features [26]. In the later phase, bone new formation leading to ankylosis can be seen. Upon induction of collagen-induced arthritis, wild-type mice showed an expected development of inflammatory signs with inflammatory symptoms in a major part of the animals at the end of the experiment. Knock-out as well as heterozygous showed slower development of inflammatory signs and none of the knock-out nor heterozygous mice developed full-blown disease (figure 4). This was confirmed by monitoring serum Matrix Metalloproteinase 3 (MMP3) levels, a marker known to be related to disease activity in CIA [16]. The MMP3 levels were significantly higher in WT compared to KO mice. Moreover, IL6 levels showed a lower trend in KO mice, though not statistically significant at the 0,05 level (p=0,09).

Structural damage was evaluated by histological sectioning of knee joints and SafraninO staining. Joint inflammation and destruction was evaluated by scoring several pathologic features. Synovial infiltration and cartilage destruction were significantly reduced in KO mice, after correcting for inter-cage variation (figure 5). This clearly indicates reduced joint inflammation and destruction in GDF15 null (KO) mice.

### 2.4 In vitro stimulation experiments of synovial fibroblasts confirm the key role of GDF15 in secretion of inflammatory mediators.

TNFa is a well-known mediator of joint inflammation and destruction. This is reflected by the therapeutic use of blocking antibodies (anti-TNF) to treat joint inflammation. Synovial fibroblasts from wild-type (WT) and GDF15 null mice (KO) were stimulated with TNFa to mimic inflammatory conditions. As expected, TNF induces IL6 expression. However, levels of IL6 are reduced in synovial fibroblasts derived from GDF15 null mice, further confirming the protective role of reduced GDF15 levels in joint inflammation (figure 7). This effect is even more pronounced under hypoxic conditions, induced by DMOG, which are highly relevant in an inflammatory joint.

### 2.5 Addition of anti-GDF15 antibodies results in an improved clinical score in a mouse CAIA model.

The CAIA model induced by type II-collagen-specific antibodies is characterized by broad articular inflammation and synovitis. As early as 7 days after immunisation with a cocktail of monoclonal anti-collagen antibodies, CAIA mice exhibit enthesitis which eventually evolves into a destructive polyarthritis. Osteophyte formation occurs after resolution of the inflammatory phase. In said remission phase, bone new formation is known to occur at insertion sites of the tendons in the foot [32; 33]. In the CAIA model, disease activity is assessed by measuring inflammation swelling in the affected joints (paw volume or thickness) over time. Clinical scoring is accomplished as e.g. described in Seeuws S. et al., 2010 [31].
A. Animals receiving no anti-GDF15 antibody developed maximum inflammation at day 3. Treated animals not only show a lower maximum score but are also characterized by a lower incidence at maximal inflammation and a more rapid decrease in incidence as well as in average clinical score, which demonstrates the therapeutic efficacy (figures 8, 9 and 10). Micro Computed Tomography slices of tibia indicate a higher cortical porosity in severe arthritic mice. Additionally the tibia show thickening of the cortex by new bone apposition. Both features (cortical porosity and cortical thickening) were less pronounced in comparable arthritic mice treated with anti-GDF15 antibody (figure 13A).
B. An anti-GDF15 antibody or an irrelevant antibody of the same isotype was given daily to the mice. C-terminal telopeptide of type II collagen, a marker of cartilage degradation was significantly lower in mice receiving anti-GDF15 treatment (figure 12A). Consistent results were obtained when knees of those mice were histologically scored for proteoglycan depletion and cartilage degradation (figure 12B). Furthermore also bone destruction was affected by anti-GDF15 treatment as C-terminal telopeptide of type I collagen (CTX-I), a marker of bone resorption was significantly lower in diseased mice treated with anti-GDF15 antibody (figure 13B).

The hallmark of SpA, bone new formation, occurs in this mouse model after the inflammatory phase. To test whether anti-GDF15 treatment has an effect on bone new formation in the remodeling phase, an additional group of mice was daily injected from day 13 on. Apparently, antibody treatment in this phase has a significantly decreasing effect on the bone new formation marker P1NP in serum, compared to antibody treatment in the inflammatory phase from day 0 to day 13 (figure 14A). These results are confirmed by microCT as the normally occurring bone new formation at a typical enthesial site on the foot in an isotype treated mouse is absent in the foot of an anti-GDF15 antibody treated mouse with comparable clinical score (figure 14B).
It can be concluded that treatment with anti-GDF15 antibodies abolishes bone and cartilage degradation and bone new formation in a mouse model for SpA.

### Conclusion

In the current invention, we demonstrated several novel and unexpected findings with regard to GDF15 biology in inflammatory rheumatic diseases. Our results indicate that steady state levels of GDF15 markedly differ across various inflammatory rheumatic diseases, independently of an acute phase response. Notably, serum levels of GDF15 in SpA patients are near normal, whereas levels in other inflammatory rheumatic diseases such as SLE and RA were elevated versus controls. Thus, we identified a new serum marker that is differentially expressed in SpA compared to RA and other inflammatory rheumatic disorders, an observation that appears to be not directly associated to an acute phase response.

Of particular interest was the observation that synovial fluid levels in SpA patients were markedly elevated compared to serum. Moreover, in SpA patients, GDF15 synovial fluid levels correlated well with disease activity and joint destruction parameters, pointing to a role for GDF15 in joint pathogenesis, not only in RA but also in SpA patients, including Psoriatic Arthritis patients. Even though the precise role of GDF15 in joint homeostasis is currently unclear, it should be noted that other members of the TGFβ family, have been linked to the modulation of the immune system as well as to bone formation, a process that differentiates RA from SpA [18,19]. The elevated synovial fluid levels of GDF15 might be related to the remodeling features that typically are associated with SpA associated arthritis. This hypothesis is further confirmed by the finding that genetic deletion of GDF15 in mice results in elevated Dickkopf protein 1 (Dkk1) levels. Dkk1 is a major modulator of joint remodeling by inhibiting Wnt signaling, and was previously related to ankylosis features of SpA [20]. In synovial fluid, GDF15 levels were also increased in OA; a disease also characterized by prominent joint remodeling.

Given the observed different disease phenotype in collagen-induced arthritis and the reduced expression of IL6 by GDF15 knock-out synovial fibroblasts under inflammatory conditions, it is clear that GDF15 acts as an active modulator of joint inflammation and that reducing GDF15 levels actively modulates the joint inflammation phenotype in vivo.

As it is known that CCR2/MCP1 axis contribute to synovial inflammation in animal models, the observed upregulated GDF15 concentrations in arthritic joints might have important functional consequences in the interplay between stromal tissues and the immune system. Indeed, we found that GDF15 is, at least in part, produced in the joint, more specifically in the synovial tissue. Overall, we show that GDF15, a protein secreted by the connective tissue, is an active mediator of joint inflammation and might contribute to inflammatory and remodeling features in arthritic joint diseases. Moreover, the clinical efficacy of an anti-GDF15 ligand has been demonstrated for the first time in the present invention.

### REFERENCES

1. Bootcov MR, Bauskin AR, Valenzuela SM, Moore AG, Bansal M, He XY, Zhang HP, Donnellan M, Mahler S, Pryor K et al: MIC-1, a novel macrophage inhibitory cytokine, is a divergent member of the TGF-beta superfamily. Proc Natl Acad Sci U S A 1997, 94(21):11514-11519.
2. Bottner M, Laaff M, Schechinger B, Rappold G, Unsicker K, Suter-Crazzolara C: Characterization of the rat, mouse, and human genes of growth/differentiation factor-15/macrophage inhibiting cytokine-1 (GDF-15/MIC-1). Gene 1999, 237(1):105-111.
3. Paralkar VM, Vail AL, Grasser WA, Brown TA, Xu H, Vukicevic S, Ke HZ, Qi H, Owen TA, Thompson DD: Cloning and characterization of a novel member of the transforming growth factor-beta/bone morphogenetic protein family. J Biol Chem 1998, 273(22):13760-13767.
4. Mimeault M, Batra SK: Divergent molecular mechanisms underlying the pleiotropic functions of macrophage inhibitory cytokine-1 in cancer. J Cell Physiol, 224(3):626-635.
5. Wollert KC, Kempf T, Peter T, Olofsson S, James S, Johnston N, Lindahl B, Horn-Wichmann R, Brabant G, Simoons ML et al: Prognostic value of growth-differentiation factor-15 in patients with non-ST-elevation acute coronary syndrome. Circulation 2007, 115(8):962-971.
6. Khan SQ, Ng K, Dhillon O, Kelly D, Quinn P, Squire IB, Davies JE, Ng LL: Growth differentiation factor-15 as a prognostic marker in patients with acute myocardial infarction. Eur Heart J 2009, 30(9):1057-1065.
7. Taddei S, Virdis A: Growth differentiation factor-15 and cardiovascular dysfunction and disease: malefactor or innocent bystander? Eur Heart J, 31(10):1168-1171.
8. de Jager SC, Bermudez B, Bot I, Koenen RR, Bot M, Kavelaars A, de Waard V, Heijnen CJ, Muriana FJ, Weber C et al: Growth differentiation factor 15 deficiency protects against atherosclerosis by attenuating CCR2-mediated macrophage chemotaxis. J Exp Med.
9. Brown DA, Moore J, Johnen H, Smeets TJ, Bauskin AR, Kuffner T, Weedon H, Milliken ST, Tak PP, Smith MD et al: Serum macrophage inhibitory cytokine 1 in rheumatoid arthritis: a potential marker of erosive joint destruction. Arthritis Rheum 2007, 56(3):753-764.
10. Vander Cruyssen B, Cantaert T, Nogueira L, Clavel C, De Rycke L, Dendoven A, Sebag M, Deforce D, Vincent C, Elewaut D et al: Diagnostic value of anti-human citrullinated fibrinogen ELISA and comparison with four other anti-citrullinated protein assays. Arthritis Res Ther 2006, 8(4):R122.
11. Arnett FC, Edworthy SM, Bloch DA, McShane DJ, Fries JF, Cooper NS, Healey LA, Kaplan SR, Liang MH, Luthra HS et al: The American Rheumatism Association 1987 revised criteria for the classification of rheumatoid arthritis. Arthritis Rheum 1988, 31(3):315-324.
12. Dougados M, van der Linden S, Juhlin R, Huitfeldt B, Amor B, Calin A, Cats A, Dijkmans B, Olivieri I, Pasero G et al: The European Spondylarthropathy Study Group preliminary criteria for the classification of spondylarthropathy. Arthritis Rheum 1991, 34(10):1218-1227.
13. Vander Cruyssen B, Van Looy S, Wyns B, Westhovens R, Durez P, Van den Bosch F, Veys EM, Mielants H, De Clerck L, Peretz A et al: DAS28 best reflects the physician's clinical judgment of response to infliximab therapy in rheumatoid arthritis patients: validation of the DAS28 score in patients under infliximab treatment. Arthritis Res Ther 2005, 7(5):R1063-1071.
14. Lambrecht S, Verbruggen G, Elewaut D, Deforce D: Differential expression of alphaB-crystallin and evidence of its role as a mediator of matrix gene expression in osteoarthritis. Arthritis Rheum 2009, 60(1):179-188.
15. Hsiao EC, Koniaris LG, Zimmers-Koniaris T, Sebald SM, Huynh TV, Lee SJ: Characterization of growth-differentiation factor 15, a transforming growth factor beta superfamily member induced following liver injury. Mol Cell Biol 2000, 20(10):3742-3751.
16. Seeuws S, Jacques P, Van Praet J, Drennan M, Coudenys J, Decruy T, Deschepper E, Lepescheux L, Pujuguet P, Oste L et al: A multiparameter approach to monitor disease activity in collagen-induced arthritis. Arthritis Res Ther, 12(4):R160.
17. Moore AG, Brown DA, Fairlie WD, Bauskin AR, Brown PK, Munier ML, Russell PK, Salamonsen LA, Wallace EM, Breit SN: The transforming growth factor-ss superfamily cytokine macrophage inhibitory cytokine-1 is present in high concentrations in the serum of pregnant women. J Clin Endocrinol Metab 2000, 85(12):4781-4788.
18. Singh R, Aggarwal A, Misra R: Th1/Th17 cytokine profiles in patients with reactive arthritis/undifferentiated spondyloarthropathy. J Rheumatol 2007, 34(11):2285-2290.
19. Braun J, Bollow M, Neure L, Seipelt E, Seyrekbasan F, Herbst H, Eggens U, Distler A, Sieper J: Use of immunohistologic and in situ hybridization techniques in the examination of sacroiliac joint biopsy specimens from patients with ankylosing spondylitis. Arthritis Rheum 1995, 38(4):499-505.
20. Diarra D, Stolina M, Polzer K, Zwerina J, Ominsky MS, Dwyer D, Korb A, Smolen J, Hoffmann M, Scheinecker C et al: Dickkopf-1 is a master regulator of joint remodeling. Nature medicine 2007, 13(2):156-163.
21. Wakchoure S, Swain TM, Hentunen TA, Bauskin AR, Brown DA, Breit SN, Vuopala KS, Harris KW, Selander KS: Expression of macrophage inhibitory cytokine-1 in prostate cancer bone metastases induces osteoclast activation and weight loss. Prostate 2009, 69(6):652-661.
22. Vanhara P, Lincova E, Kozubik A, Jurdic P, Soucek K, Smarda J: Growth/differentiation factor-15 inhibits differentiation into osteoclasts--a novel factor involved in control of osteoclast differentiation. Differentiation 2009, 78(4):213-222.
23. Vandooren B, Noordenbos T, Ambarus C, Krausz S, Cantaert T, Yeremenko N, Boumans M, Lutter R, Tak PP, Baeten D: Absence of a classically activated macrophage cytokine signature in peripheral spondylarthritis, including psoriatic arthritis. Arthritis Rheum 2009, 60(4):966-975.
24. Fairlie WD, Moore AG, Bauskin AR, Russell PK, Zhang HP, Breit SN: MIC-1 is a novel TGF-beta superfamily cytokine associated with macrophage activation. J Leukoc Biol 1999, 65(1):2-5.
25. Breit SN, Johnen H, Cook AD, Tsai VW, Mohammad MG, Kuffner T, Zhang HP, Marquis CP, Jiang L, Lockwood G, Lee-Ng M, Husaini Y, Wu L, Hamilton JA, Brown DA. The TGF-β superfamily cytokine, MIC-1/GDF15: a pleotrophic cytokine with roles in inflammation, cancer and metabolism. Growth Factors. 2011, Oct;29(5): 187-95.
26. Courtenay JS, Dallman MJ, Dayan AD, Martin A, Mosedale B: Immunisation against heterologous type II collagen induces arthritis in mice. Nature 1980, 283:666-668.
27. Dimitrov, D.S. (2009) Engineered CH2 domains (nanoantibodies). MABS 1(1), 26-28.
28. Khaw, B. A. et al. J. Nucl. Med. 23:1011-1019 (1982).
29. Rousseaux et al. Methods Enzymology, 121:663-69, Academic Press, 1986.
30. Tramontano A, Bianchi E, Venturini S, Martin F, Pessi A, Sollazzo M. The making of the minibody: an engineered beta-protein for the display of conformationally constrained peptides. J Mol Recognit. 1994 Mar;7(1):9-24.
31. A multiparameter approach to monitor disease activity in collagen-induced arthritis. Seeuws S, Jacques P, Van Praet J, Drennan M, Coudenys J, Decruy T, Deschepper E, Lepescheux L, Pujuguet P, Oste L, Vandeghinste N, Brys R, Verbruggen G, Elewaut D. Arthritis Res Ther. 2010;12(4):R160.
32. Jacques, P., Lambrecht, S., Verheugen, E., Pauwels, E., Kollias, G., Armaka, M., Verhoye, M., Van der Linden, A., Achten, R., Lories, R.J., Elewaut, D. (2014). "Proof of concept: enthesitis and new bone formation in spondyloarhtritis are driven by mechanical strain and stromal cells." Ann Rheum Dis. 73(2): 437-45.
33. Sherlock JP, Joyce-Shaikh B, Turner SP, Chao CC, Sathe M, Grein J, Gorman DM, Bowman EP, McClanahan TK, Yearley JH, Eberl G, Buckley CD, Kastelein RA, Pierce RH, Laface DM, Cua DJ. IL-23 induces spondyloarthropathy by acting on ROR-γt+ CD3+CD4-CD8- entheseal resident T cells. Nat Med. 2012 Jul 1;18(7):1069-76.
34. Vlassenbroeck J, Dierick M, Masschaele B, Cnudde V, Van Hoorebeke L, Jacobs P. 2007. Instruments and Methods in Physics Research Section A: Accelerators, Spectrometers, Detectors and Associated Equipment 580: 442-445. Software tools for quantification of X-ray microtomography at the UGCT; Nuclear

## Claims

1. An antibody binding Growth Differentiation Factor 15 (GDF15), or a GDF15 binding antibody fragment thereof, for use in treating rheumatoid arthritis (RA), spondyloarthritis (SpA), psoriatic arthritis or osteoarthritis (OA).

2. The antibody for use according to claim 1, wherein said antibody is a chimeric antibody, a humanized antibody, a human engineered antibody, a human antibody, or a single chain antibody; or wherein the antibody fragment is a Fab fragment, a Fab' fragment, a F(ab')2 fragment, a Fd fragment, a Fv fragment, a scFv fragment, heavy chain antibodies (hcAb), a minibody, a variable domain of camelid heavy chain antibody (VHH), a variable domain of the new antigen receptor (VNAR) or an engineered CH2 domain.

3. The antibody for use according to claim 1, wherein the antibody is the monoclonal antibody produced by the hybridoma cell line deposited at BCCM on 30 September, 2014, and given deposit number LMBP 10815CB.

4. A pharmaceutical composition comprising an antibody binding GDF15, or a GDF15 binding antibody fragment thereof, and a pharmaceutically acceptable excipient for use in treating rheumatoid arthritis (RA), spondyloarthritis (SpA), psoriatic arthritis or osteoarthritis (OA).

5. The pharmaceutical composition for use according to claim 4, wherein the GDF15 binding antibody, or the GDF15 binding antibody fragment thereof, is a chimeric antibody, a humanized antibody, a human engineered antibody, a human antibody, or a single chain antibody; or wherein the antibody fragment is a Fab fragment, a Fab' fragment, a F(ab')2 fragment, a Fd fragment, a Fv fragment, a scFv fragment, heavy chain antibodies (hcAb), a minibody, a variable domain of camelid heavy chain antibody (VHH), a variable domain of the new antigen receptor (VNAR) or an engineered CH2 domain.

6. The pharmaceutical composition for use according to claim 4, wherein the antibody is the monoclonal antibody produced by hybridoma cell line deposited at BCCM on 30 September, 2014, and given deposit number LMBP 10815CB.

## Patentansprüche

1. Antikörper, der Wachstumsdifferenzierungsfaktor 15 (GDF15) bindet, oder ein GDF15 bindendes Antikörperfragment davon zur Verwendung beim Behandeln von rheumatoider Arthritis (RA), Spondyloarthritis (SpA), psoriatischer Arthritis oder Osteoarthritis (OA).

2. Antikörper zur Verwendung nach Anspruch 1, wobei es sich bei dem Antikörper um einen chimären Antikörper, einen humanisierten Antikörper, einen humanen konstruierten Antikörper, einen humanen Antikörper oder einen Einzelkettenantikörper handelt; oder wobei es sich bei dem Antikörperfragment um ein Fab-Fragment, ein Fab'-Fragment, ein F(ab')2-Fragment, ein Fd-Fragment, ein Fv-Fragment, ein scFv-Fragment, Schwere-Ketten-Antikörper (hcAb), einen Minibody, eine variable Domäne eines Kamelid-Schwere-Ketten-Antikörpers (VHH), eine variable Domäne des neuen Antigenrezeptors (VNAR) oder eine konstruierte CH2-Domäne handelt.

3. Antikörper zur Verwendung nach Anspruch 1, wobei der Antikörper der monoklonale Antikörper ist, der von der Hybridomzelllinie produziert wird, der bei der BCCM am 30. September 2014 hinterlegt wurde und dem die Hinterlegungsnummer LMBP 10815CB gegeben wurde.

4. Pharmazeutische Zusammensetzung, die einen Antikörper, der GDF15 bindet, oder ein GDF15 bindendes Antikörperfragment davon und einen pharmazeutisch akzeptablen Hilfsstoff umfasst, zur Verwendung beim Behandeln von rheumatoider Arthritis (RA), Spondyloarthritis (SpA), psoriatischer Arthritis oder Osteoarthritis (OA).

5. Pharmazeutische Zusammensetzung zur Verwendung nach Anspruch 4, wobei es sich bei dem GDF15 bindenden Antikörper oder das GDF15 bindende Antikörperfragment davon um einen chimären Antikörper, einen humanisierten Antikörper, einen humanen konstruierten Antikörper, einen humanen Antikörper oder einen Einzelkettenantikörper handelt; oder wobei es sich bei dem Antikörperfragment um ein Fab-Fragment, ein Fab'-Fragment, ein F(ab')2-Fragment, ein Fd-Fragment, ein Fv-Fragment, ein scFv-Fragment, Schwere-Ketten-Antikörper (hcAb), einen Minibody, eine variable Domäne eines Kamelid-Schwere-Ketten-Antikörpers (VHH), eine variable Domäne des neuen Antigenrezeptors (VNAR) oder eine konstruierte CH2-Domäne handelt.

6. Pharmazeutische Zusammensetzung zur Verwendung nach Anspruch 4, wobei der Antikörper der monoklonale Antikörper ist, der von einer Hybridomzelllinie produziert wird, der bei der BCCM am 30. September 2014 hinterlegt wurde und dem die Hinterlegungsnummer LMBP 10815CB gegeben wurde.

## Revendications

1. Anticorps se liant au facteur 15 de croissance et de différenciation (GDF15), ou fragment d'anticorps se liant à GDF15 de celui-ci, destiné à être utilisé dans le traitement de la polyarthrite rhumatoïde (PR), la spondylarthrite (SpA), l'arthrite psoriasique ou l'ostéoarthrite (OA).

2. Anticorps destiné à être utilisé selon la revendication 1, dans lequel ledit anticorps est un anticorps chimère, un anticorps humanisé, un anticorps humain créé par génie génétique, un anticorps humain ou un anticorps à chaîne unique ; ou dans lequel le fragment d'anticorps est un fragment Fab, un fragment Fab', un fragment F(ab')2, un fragment Fd, un fragment Fv, un fragment scFv, des anticorps à chaîne lourde (hcAb), un minicorps, un domaine variable d'anticorps à chaîne lourde de camélidé (VHH), un domaine variable du nouveau récepteur d'antigène (VNAR) ou un domaine CH2 créé par génie génétique.

3. Anticorps destiné à être utilisé selon la revendication 1, dans lequel l'anticorps est l'anticorps monoclonal produit par la lignée cellulaire d'hybridomes déposée au BCCM le 30 septembre 2014 et à laquelle le numéro de dépôt LMBP 10815CB est donné.

4. Composition pharmaceutique comprenant un anticorps se liant à GDF15, ou un fragment d'anticorps se liant à GDF15 de celui-ci, et un excipient pharmaceutiquement acceptable destinée à être utilisée dans le traitement de la polyarthrite rhumatoïde (PR), la spondylarthrite (SpA), l'arthrite psoriasique ou l'ostéoarthrite (OA).

5. Composition pharmaceutique destinée à être utilisée selon la revendication 4, dans laquelle l'anticorps se liant à GDF15, ou le fragment d'anticorps se liant à GDF15 de celui-ci, est un anticorps chimère, un anticorps humanisé, un anticorps humain créé par génie génétique, un anticorps humain ou un anticorps à chaîne unique ; ou dans laquelle le fragment d'anticorps est un fragment Fab, un fragment Fab', un fragment F(ab')2, un fragment Fd, un fragment Fv, un fragment scFv, des anticorps à chaîne lourde (hcAb), un minicorps, un domaine variable d'anticorps à chaîne lourde de camélidé (VHH), un domaine variable du nouveau récepteur d'antigène (VNAR) ou un domaine CH2 créé par génie génétique.

6. Composition pharmaceutique destinée à être utilisée selon la revendication 4, dans laquelle l'anticorps est l'anticorps monoclonal produit par la lignée cellulaire d'hybridomes déposée au BCCM le 30 septembre 2014 et à laquelle le numéro de dépôt LMBP 10815CB est donné.


### SEQUENCE LISTING

<110> Universiteit Gent
<120> A METHOD OF TREATING JOINT DISEASE
<130> P2014/017PCT
<160> 1
<170> PatentIn version 3.3
<210> 1
   <211> 132
   <212> PRT
   <213> Mus Musculus
<400> 1
   Ala His His His His His His Pro Gly Gly Pro Gly Ser Asp Glu Leu
   1               5                   10                  15
   Asp Ser Ala His Ala His Pro Arg Asp Ser Cys Pro Leu Gly Pro Gly
               20                  25                  30
   Arg Cys Cys His Leu Glu Thr Val Gln Ala Thr Leu Glu Asp Leu Gly
           35                  40                  45
   Trp Ser Asp Trp Val Leu Ser Pro Arg Gln Leu Gln Leu Ser Met Cys
       50                  55                  60
   Val Gly Glu Cys Pro His Leu Tyr Arg Ser Ala Asn Thr His Ala Gln
   65                  70                  75                  80
   Ile Lys Ala Arg Leu His Gly Leu Gln Pro Asp Lys Val Pro Ala Pro
                   85                  90                  95
   Cys Cys Val Pro Ser Ser Tyr Thr Pro Val Val Leu Met His Arg Thr
               100                 105                 110
   Asp Ser Gly Val Ser Leu Gln Thr Tyr Asp Asp Leu Val Ala Arg Gly
           115                 120                 125
   Cys His Cys Ala
       130